(19) Europäisches Patentamt
European Patent Office
Office européen des brevets

(11) Veröffentlichungsnummer: **0 169 571**
**B1**

(12) # EUROPÄISCHE PATENTSCHRIFT

(45) Veröffentlichungstag der Patentschrift: **09.03.88**

(21) Anmeldenummer: **85109383.1**

(22) Anmeldetag: **26.07.85**

(51) Int. Cl.[4]: **C 07 C 59/64,** C 07 C 57/42, C 07 C 69/618, C 07 C 69/732, C 07 C 69/734, C 07 C 51/353, C 07 C 67/343, C 07 C 101/453, C 07 C 99/00, C 07 C 43/20, C 07 C 47/575

(54) **Phenylnonatetraensäurederivate, ihre Herstellung und pharmazeutische Präparate mit diesen Derivaten.**

(30) Priorität: **27.07.84 US 635100**

(43) Veröffentlichungstag der Anmeldung: **29.01.86 Patentblatt 86/5**

(45) Bekanntmachung des Hinweises auf die Patenterteilung: **09.03.88 Patentblatt 88/10**

(84) Benannte Vertragsstaaten: **AT BE CH DE FR GB IT LI LU NL SE**

(56) Entgegenhaltungen:
**EP - A - 0 059 365**
**DE - A - 2 414 619**
**FR - A - 2 285 864**
**GB - A - 1 101 396**

(73) Patentinhaber: **F. HOFFMANN-LA ROCHE & CO. Aktiengesellschaft, CH-4002 Basel (CH)**

(72) Erfinder: **Aig, Edward Roy, 13-05 Alexander Avenue, Fair Lawn, N.J. (US)**
Erfinder: **Coffey, John William, 152 Washington Avenue, West Caldwell, N.J. (US)**
Erfinder: **Lovey, Allen John, 65 Woodside Avenue, West Caldwell, N.J. (US)**
Erfinder: **Rosenberger, Michael, 79 Arlington Avenue, Caldwell, N.J. (US)**

(74) Vertreter: **Lederer, Franz, Dr. et al, Van der Werth, Lederer & Riederer Patentanwälte Lucile-Grahn-Strasse 22, D-8000 München 80 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft Verbindungen der Formel

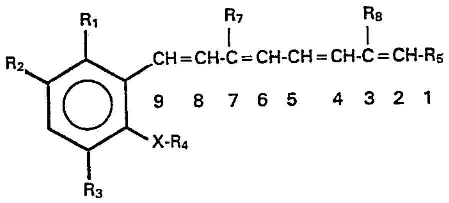

I

worin

$R_1$ Wasserstoff, Niederalkyl, Chlor, Fluor oder Trifluormethyl; $R_2$ Wasserstoff, Niederalkoxy, Trifluormethylniederalkoxy, Hydroxy, Niederalkyl, Chlor, Trifluormethyl oder Fluor; $R_3$ Wasserstoff, Niederalkyl, Chlor oder Fluor; $R_4$ Alkyl mit 4-10 C-Atomen oder $-CH_2(CH_2))_nCH_2OH$; X $-CH(R_{10})O-$, $-CH(R_{10})-$, -O-, $-C(R_{10})=CH-$ oder $-N(R_{10})-$; $R_5$ $COOR_9$; n 6 oder 7; und $R_7$, $R_8$, $R_9$ und $R_{10}$ Nideralkyl oder Wasserstoff bedeuten und Salze davon, wenn $R_9$ Wasserstoff ist.

Die Erfindung betrifft weiterhin ein Verfahren zur Herstellung von Verbindungen der Formel I und pharmazeutische Kompositionen die solche Verbindungen enthalten.

In den Formelblättern 1, 2, 3, 4, 5, 6 und 7 sind die Verfahrensschritte zur Herstellung der Verbindungen der Formel I schematisch angegeben.

Der hier verwendete Ausdruck «Halogen» umfasst alle vier Halogene, d.h. Chlor, Brom, Jod und Fluor, wobei Chlor und Brom bevorzugt sind. Der Ausdruck «Niederalkyl» bezeichnet geradkettige und verzweigte Alkylgruppen mit 1-7 C-Atomen. Bevorzugte Niederealkylgruppen sind Methyl, Äthyl, Isopropyl und n-Butyl, wobei Methyl und Äthyl besonders bevorzugt sind. Der Ausdruck «Niederalkoxy» bezeichnet Niederealkoxygruppen mit 1-7 C-Atomen wie Methoxy, Äthoxy, Isopropoxy und Isobutoxy. Der Ausdruck «Trifluormethylniederalkoxy» bezeichnet eine wie oben definierte Niederalkoxygruppe, die durch Trifluormethyl substituiert ist. Der Ausdruck «Alkyliden» bezeichnet einen gesättigten aliphatischen Kohlenwasserstoffrest dessen terminales C-Atom zwei freie Valenzen aufweist.

Der Ausdruck «Aryl» bezeichnet einkernige aromatische Kohlenwasserstoffgruppen, die unsubstituiert oder in einer oder mehreren Stellungen durch Niederalkylgruppen substituiert sein können, wie Phenyl oder Tolyl; und mehrkernige aromatische Gruppen, die unsubstituiert oder in einer oder mehreren Stellungen durch eine Niederalkylgruppe substituiert sein können wie Naphthyl, Phenanthryl oder Anthryl. Die bevorzugte Arylgruppe ist Phenyl.

In einer Ausführungsform der Verbindung der Formel I ist X -O-, $R_7$ und $R_8$ Niederalkyl, vorzugsweise Methyl. In einer anderen bevorzugten Ausführungsform ist $R_4$ $-(CH_2)y$ H wobei y 6-9 insbesondere 8-9, vorzugsweise 9 ist. In dieser Ausführungsform sind $R_1$, $R_2$ und $R_3$ vorzugsweise Wasserstoff oder $R_1$ und $R_3$ sind Wasserstoff und $R_2$ Niederalkoxy wie Methoxy oder Äthoxy. Andererseits kann in dieser Ausführungsform $R_2$ und $R_3$ Wasserstoff sein, $R_1$ Chlor oder Fluor oder $R_1$ und $R_2$ sind Wasserstoff und $R_3$ ist Chlor oder Fluor.

Eine weitere bevorzugte Gruppe von Verbindungen der Formel I sind solche, in denen $R_4$ $-CH_2(CH_2)_n-CH_2OH$ ist. Hierbei sind $R_1$, $R_2$ und $R_3$ vorzugsweise Wasserstoff oder $R_1$ ist Chlor oder Fluor und $R_2$ und $R_3$ sind Wasserstoff. Andererseits sind von diesen Verbindungen solche bevorzugt, in denen $R_1$ und $R_3$ Wasserstoff und $R_2$ Niederalkoxy insbesondere Methoxy oder Äthoxy sind. Weiterhin sind solche Verbindungen bevorzugt, in denen $R_1$ und $R_2$ Wasserstoff sind und $R_3$ Chlor oder Fluor ist.

Die Erfindung bezieht sich auch auf Verbindungen der Formel I mit pharmazeutisch anwendbaren, nicht toxischen anorganischen oder organischen Basen, z.B. Alkalimetall, Erdalkalimetallsalze. Bevorzugte Salze sind das Natrium-, Kalium-, Magnesium- oder Calciumsalz, wie auch Salze mit Ammoniak oder geeigneten nicht toxischen Aminen wie Niederalkylaminen, beispielsweise Triäthylamin; Hydroxyniederalkylaminen, beispielsweise 2-Hydroxyäthylamin, bis-(1-Hydroxyäthyl)amin oder tris-(2-Hydroxyäthylamin; Cycloalkylaminen, beispielsweise Dicyclohexylamin; oder Benzylaminen, beispielsweise N,N'-Dibenzyläthylendiamin und Dibenzylamin. Diese Salze können durch Behandlung der Verbindung der Formel I in denen $R_9$ Wasserstoff ist mit anorganischen oder organischen Basen nach herkömmlichen Methoden hergestellt werden.

Die Verbindungen der Formel I und deren Salze sind wirksam als Mittel zur Behandlung von rheumatoider Arthritis und verwandter Erkrankungen wie Osteoarthritis. Dabei vermindern diese Verbindungen die durch die Krankheit hervorgerufenen Schäden an den Knochengelenken wie auch die Entzündung und die dadurch hervorgerufenen Schmerzen.

Die Verbindungen der Formel I und deren Salze sind weiterhin zur Behandlung von Krankheiten geeignet, die durch übermässige Immunaktivität hervorgerufen werden wie Transplantationsautoimmunität, Autoimmunerkrankung und graft versus host disease. Die erfindungsgemässen Verbindungen sind unerwartet untoxisch, wie aus dem Umstand zu ersehen ist, dass die Verbindung (all-E)-8-[2-(Nonyloxy)-phenyl]-3,7-dimethyl-2,4,6,8-nonatetraensäure bei Mäusen eine $LD_{50}$ über 1000 mg/kg i.p. und p.o. besitzt.

Die antiarthritische Wirksamkeit der erfindungsgemässen Verbindungen wird aus den Testresultaten ersichtlich, die an Ratten im Testsystem Chronische Adjuvansarthritis (Billingham und Davies «Handbook of Experimental Pharmacology» (Herausgeber J.R. Vane und S.H. Ferreira Volumen 50/II, pg. 108-144, Springer-Verlag, Berlin, 1979) erhalten wurden.

Bei diesem Verfahren wird eine Adjuvans-Arthritis durch subplantare Injektion von 0,05 ml Adjuvans (eine Suspension von durch Hitze abgetöteten, getrockneten Mycobacterium butyricum, 0,5% (Gew.-Vol.) in Mineralöl, das 0,2% Digitonin enthält) in die rechte Hinterpfote von männlichen Charles River Lewis-ratten (120-140 g) erzeugt. Das Volumen der beiden Hinterpfoten wird unmittelbar nach Injektion

des Adjuvans (Tag 0) gemessen. Das Pfotenvolumen wird in Abständen 3-7 Tagen gemessen, um die Schwellung der arthritischen Pfote die durch die Entzündung erzeugt wird, zu ermitteln.

Die Testsubstanzen wurden einmal täglich verabreicht beginnend mit dem Tag der Injektion des Adjuvans, wobei Tween 80 (Polyoxyäthylensorbitanmonooleat) in einer Menge von 0,25 ml/100 g Körpergewicht als Vehikel verwendet wurde. Arthritische Kontrollratten erhielten tägliche Dosen des Vehikels allein. Am 23-25 Versuchstag wurden die Ratten getötet, das Plasma gesammelt und die Plasmafibrinogenspiegel bestimmt (Ammoniumsulfatturbidimetrische Methode); Exner et al., Amer. J. Clin. Path. 71: 521-527 (1979). Die antiinflammatorische Wirksamkeit der Testverbindungen wurde durch Vergleich des Ausmasses der Pfotenschwellung (Pfotenvolumen an einem bestimmten Tag minus Pfotenvolumen am Versuchsbeginn) bei den behandelten Tieren und denen, die nur das Vehikel erhalten hatten bestimmt. Die Abnahme des Plasmafibrinogenspiegels wurde ebenfalls herangezogen um die antiinflammatorische Aktivität zu messen.

In Tabelle I sind die Resultate der Versuche mit verschiedenen erfindungsgemässen Verbindungen im Vergleich mit Indomethacin und 13-cis-Vitamin A Säure angeführt.

TABELLE 1

| Vehikel | Dosis p.o. µMol/Kg | % Reduktion des Pfotenvolumens am 23. Tag | | Plasma-fibrinogen % Abnahme | Körpergewicht Änderung (g) |
|---|---|---|---|---|---|
| | | rechts | links | | |
| | -- | --- | ---- | ---- | 28 - 31 |
| Indomethacin | 3 | -66 | -67 | 30 | +46 |
| 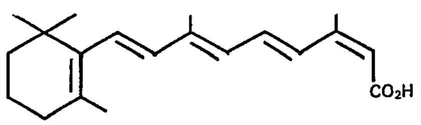 | 60 | -33 | -49 | 18 | -7 |
| 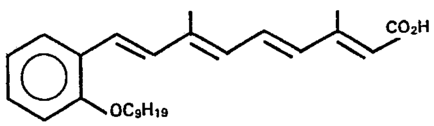 | 76 | -43 | -58 | 55 | +27 |
| 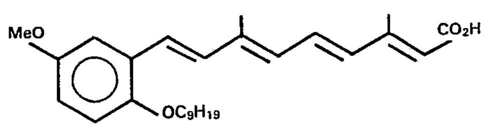 | 75 | -27 | -24 | 21 | +27 |
| 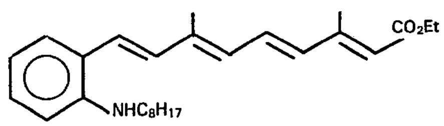 | 75 | -53 | -53 | | +24 |
| 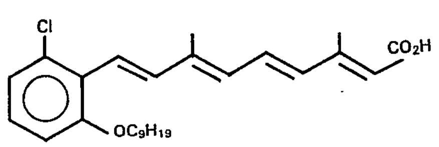 | 75 | -29 | -32 | | +35 |
| 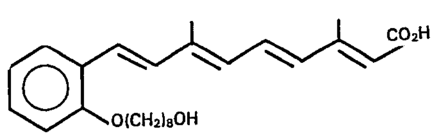 | 95 | -37 | -49 | 35 | +27 |
| 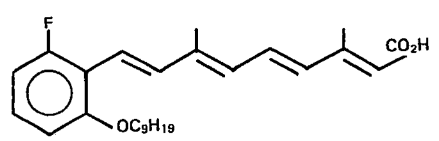 | 75 | -56 | -68 | 51 | +46 |

Wie aus der Tabelle I ersichtlich, wird die durch das Adjuvans bewirkte Schwellung durch die erfindungsgemässen Verbindungen wirksam reduziert. Die erfindungsgemässen Verbindungen vermindern weiterhin den Plasmafibrinogenspiegel, der generell mit rheumatoider Arthritis assoziiert ist. Weiterhin ist zu sehen, dass die erfindungsgemässen Verbindungen bei den angegebenen Dosierungen keine wesentliche Verminderung in der Gewichtszunahme der Tiere bewirkten. Dies ist ein Zeichen für das Fehlen toxischer Eigenschaften der erfindungsgemässen Verbindungen.

Die Verbindungen der Formel I und ihre pharmazeutisch anwendbaren Salze können in einer Vielzahl von pharmazeutischen Präparaten angewandt werden. Die Verbindungen können in solchen Präparaten in Form oraler Dosiseinheiten wie Tabletten, Pillen, Pulvern und Kapseln wie auch als Injektionslösungen, Lösungen, Suppositorien, Emulsionen, Dispersionen und in anderen geeigneten Formen angewandt werden. Die pharmazeutischen Präparate, die Verbindungen der Formel I enthalten, werden zweckmässig durch Vermischen mit einem nicht toxischen pharmazeutischen organischen oder anorganischen Träger hergestellt. Typische pharmazeutisch anwendbare Träger sind beispielsweise Wasser, Gelatine, Laktose, Stärke, Magnesiumstearat, Talk, vegetabile Öle, Polyalkylenglycole, Vaseline und andere üblicherweise angewandte pharmazeutisch akzeptable Träger. Die pharmazeutischen Präparate können auch nicht-toxische Hilfssubstanzen wie Emulgatoren, Konservierungs- und Befeuchtungsmittel enthalten; beispielsweise Sorbitanmonolaurat, Triäthanolaminoleat, Polyoxyäthylensorbitan und Dioctylnatriumsulfosuccinat.

Die täglich zu verabreichende Dosis der Verbindungen variiert mit der angewandten Verbindung, der Art der Verabreichung und der Grösse des Empfängers. Die verabreichte Dosis ist nicht an bestimmte Grenzen gebunden, sie bewegt sich üblicherweise in den für die pharmakologische Funktion der Verbindung wirksamen Mengen.

Bei der oralen Verabreichung können die Verbindungen der Formel I und ihre Salze in Dosierungen von 0,5 mg/kg pro Tag bis 100 mg/kg pro Tag verabreicht werden. Vorzugsweise verabreicht man die Verbindungen täglich an Patienten in oralen Dosiseinheiten mit täglichen Dosierungen von 1-30 mg/kg Körpergewicht, vorzugsweise 1-10 mg/kg.

Die Verbindungen der Formel I, in denen X -O- ist können aus Verbindungen der Formel

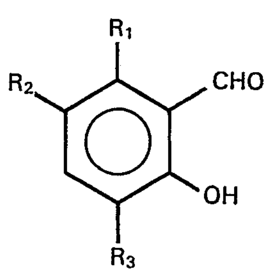

III

worin $R_1$, $R_2$ und $R_3$ die obige Bedeutungen haben, nach dem Reaktionsschema von Formelblatt I hergestellt werden.

In Formelblatt I sind n, $R_1$, $R_2$, $R_3$, $R_4$, $R_7$ und $R_8$ wie oben, $R'_9$ ist Niederalkyl, Z ist eine Abgangsgruppe, Y ist Aryl, vorzugsweise Phenyl, und Z' ist Halogen.

Die Verbindung der Formel III wird in eine Verbindung der Formel IV im Reaktionsschritt a) durch Reduktion der Aldehydgruppe zum Alkohol umgewandelt. Diese Reaktion wird mit üblichen Reduktionsmitteln, die Aldehyde zu Alkoholen umwandeln, durchgeführt. Im allgemeinen wird ein Alkalimetallborhydrid wie Natriumborhydrid als Reduktionsmittel verwendet. Hierbei können die für solche Reduktionen üblichen Verfahrensbedingungen Anwendung finden. Wenn $R_2$ Hydroxy ist, wird die Hydroxygruppe während der Reduktion der Verbindung III und der nachfolgenden Umwandlung in eine Verbindung der Formel I vorzugsweise geschützt. Jede übliche hydrolysierbare Hydroxyschutzgruppe, wie Niederalkylgruppen, kann hierbei Verwendung finden. Diese Esterschutzgruppe kann durch übliche Esterhydrolyse nach der Bildung des Wittig-Salzes der Formel XIII und XVI oder nach der Bildung des Äthers der Formel X abgespalten werden.

Die Verbindung der Formel IV wird in eine Verbindung der Formel VI in der Reaktionsstufe b) durch Behandlung der Verbindung IV mit einem Triarylphosphinhydrohalogenid umgewandelt. Dabei wird das Phosphoniumsalz der Formel VI hergestellt. Bei dieser Reaktion können alle konventionellen Methoden der Umsetzung eines Allylalkohols mit einem Triarylphosphinhydrohalogenid Anwendung finden. Das Phosphoniumsalz der Formel VI wird durch eine Wittig-Reaktion mit der Verbindung der Formel VII in Stufe c) zu einer Verbindung der Formel VIII umgesetzt. Alle bei Wittig-Reaktion üblichen Bedingungen können bei der Durchführung der Reaktion c) Anwendung finden.

Andererseits kann die Verbindung der Formel III direkt in eine Verbindung der Formel VIII durch Reaktion mit dem Phosphoniumsalz der Verbindung der Formel IX [Stufe e)] umgewandelt werden. Die Umsetzung des Phosphoniumsalzes der Formel IX mit der Verbindung der Formel III zu der Verbindung der Formel VIII wird unter den gleichen Bedingungen wie in Verbindung mit Reaktion c) beschrieben ausgeführt.

Die Verbindung der Formel VIII wird in eine Verbindung der Formel X durch Verätherung oder Alkylierung mit einer Verbindung der Formel V [Reaktion e)] umgewandelt. In der Verbindung der Formel V kann Z jede übliche Abgangsgruppe wie Mesyloxy, Tosyloxy oder ein Halogenid darstellen. Alle konventionellen Methoden zur Verätherung einer Hydroxygruppe durch Reaktion mit einem Halogenid oder einer Abgangsgruppe können bei der Durchführung der Reaktion d) angewandt werden.

In einer anderen Ausführungsform kann eine Verbindung der Formel X, in der eine Hydroxygruppe $R_2$ als hydrolysierbare Ester geschützt ist, aus einer Verbindung der Formel III durch Alkylierung oder Verätherung der Verbindung der Formel III mit der Verbindung der Formel V zu einer Verbindung der Formel XI hergestellt werden. Diese Umsetzung wird durch

Alkylieren der Verbindung der Formel III mit der Verbindung der Formel V in Stufe d) durchgeführt. In den Reaktionen f) und e) braucht eine Hydroxygruppe in einer Hydroxyalkylgruppe $R_4$ nicht geschützt zu werden. Unter den Bedingungen die in diesem Reaktionsschritt angewandt werden, reagiert die Verbindung der Formel V mit der Verbindung der Formel III oder der Verbindung der Formel VIII unter Bildung der Verbindung der Formel XI oder der Verbindung der Formel X, ohne dass die Notwendigkeit besteht, die Hydroxygruppe in der Alkylkette zu schützen. Alkylierung oder Verätherung findet direkt an der phenolischen Gruppe der Verbindung III oder VIII statt und die Hydroxygruppe in der Alkylseitenkette $R_4$ wird nicht oder kaum angegriffen. Die Verbindungen der Formel XI wird im Verfahrensschritt g) durch Reduktion in die Verbindung der Formel XII übergeführt. Hierfür können die im Zusammenhang mit Reaktionsschritt a) beschriebenen Reaktionsbedingungen angewandt werden.

Im Reaktionsschritt h) wird eine Verbindung der Formel XII in eine Verbindung der Formel XIII durch Behandlung mit einem Triarylphosphinhydrohalogenid in der im Zusammenhang mit Stufe b) beschriebenen Weise übergeführt. Die Verbindung der Formel XIII wird in eine Verbindung der Formel X durch Umsetzung mit einer Verbindung der Formel VII gemäss Reaktionsschritt i) übergeführt. Dieser Verfahrensschritt wird in der gleichen Weise durchgeführt wie im Zusammenhang mit Reaktionsschritt c) beschrieben.

Gemäss einer anderen Ausführungsform wird die Verbindung der Formel X dadurch hergestellt, dass man zunächst eine Verbindung der Formel XI in eine Verbindung der Formel XIV überführt. Die Verbindung der Formel XI wird durch Aldolkondensation mit einer Verbindung der Formel XX in eine Verbindung der Formel XIV übergeführt. Hierfür können die bekannten Methoden für Aldolkondensationen angewandt werden. Im nächsten Schritt wird die Verbindung der Formel XIV entweder durch Grignard-Reaktion mit einem Vinylmagnesiumhalogenid oder Lithiumkondensation mit Vinyllithium zu einer Verbindung der Formel XV umgesetzt. Die Reaktion von Stufe k) kann unter konventionellen Bedingungen für Lithiumkondensationen oder Grignard-Reaktionen ausgeführt werden. Die Verbindung der Formel XV wird durch Umsetzung mit einem Triarylphosphinhydrohalogenid in der im Zusammenhang mit der Reaktion b) beschriebenen Weise zu einer Verbindung der Formel XVI umgesetzt. Die Verbindung der Formel XVI wird danach im Reaktionsschritt m) mit einer Verbindung der Formel XVII in eine Verbindung der Formel X übergeführt. Die Reaktion m) wird als Standard-Wittig-Reaktion wie im Zusammenhang mit der Reaktion c) beschrieben durchgeführt. Die Verbindung der Formel X kann in die freie Säure, d.h. in die Verbindung der Formel I, worin $R_5$ COOH ist, durch Esterhydrolyse übergeführt werden. Jedes übliche Verfahren zur Esterhydrolyse liefert eine Verbindung der Formel I mit $R_5$ = COOH.

Die Verbindungen der Formel I worin X $-N(R_{10})-$ ist, werden aus Verbindungen der Formel

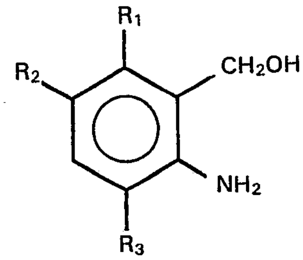

XXII

worin $R_1$, $R_2$ und $R_3$ die obige Bedeutung haben, gemäss dem Reaktionsschema in Formelblatt 2 hergestellt.

Im Reaktionsschema 2 haben $R_1$, $R_2$, $R_3$, $R_4$, $R_7$, $R_8$, $R'_9$, Z' und Y die obige Bedeutung. $R_{13}$ hat die gleiche Bedeutung wie $R_4$ mit einem Kohlenstoffatom weniger als die in $R_4$ enthaltene Alkylgruppe. Infolgedessen ist $R_{13}$ eine Alkylgruppe mit 3-9 C-Atomen oder $-CH_2(CH_2)_mCH_2OH$ wobei m eine um 1 geringere Zahl als n ist, d.h. m ist 5 oder 6. $R_{10}$ ist Wasserstoff oder Niederalkyl mit 1-7 C-Atomen und $R_{10}'$ ist eine Niederalkylgruppe mit 1-7 C-Atomen. $R_{10}''$ ist eine Niederalkylgruppe mit einem Kohlenstoff weniger als die durch $R_{10}'$ bezeichnete Alkylgruppe.

Im Reaktionsschema 2 wird die Verbindung der Formel XXII mit einem Säurechlorid der Formel XIX worin Z' Halogen ist zu einer Verbindung der Formel XXIII umgesetzt, die später entweder zu einer Verbindung der Formel XXVI oder einer Verbindung der Formel XXXI umgewandelt wird. Wenn $R_{13}$ in der Verbindung der Formel XXIX $-CH_2-(CH_2)_mCH_2OH$ ist, hat die Gegenwart der Hydroxygruppe im Substituenten $R_{13}$ keinen Einfluss auf die Reaktion. Es wurde gefunden, dass die Hydroxygruppe unangegriffen in der Reaktionsfolge von Reaktionsschema 2 bleibt.

Andererseits kann diese Hydroxygruppe in Form eines hydrolysierbaren Äthers geschützt werden. Jede konventionelle Ätherschutzgruppe kann dafür verwendet werden. Beispiele für bevorzugte Ätherschutzgruppen sind Tetrahydropyranyloxy, t-Butoxy, Triniederalkylsilyloxy z.B. Trimethylsilyloxy.

In der ersten Reaktionsstufe wird eine Verbindung der Formel XXII mit einer Verbindung der Formel XIX zu einer Verbindung der Formel XXIII umgesetzt. Für diese Umsetzung können alle bekannten Methoden zur Kondensation eines Amins mit einem Säurehalogenid angewandt werden. In der nächsten Stufe wird die Verbindung der Formel XXIII im Reaktionsschritt n) in eine Verbindung der Formel XXIV durch Behandlung mit einem Reduktionsmittel übergeführt. Jedes konventionelle Alkalimetallaluminiumhydrid kann dafür Verwendung finden, wobei Lithiumaluminiumhydrid bevorzugt ist. Die Reduktion mit dem Alkalimetallaluminiumhydrid kann unter den üblichen Bedingungen durchgeführt werden.

Die Verbindung der Formel XXIV kann über das Zwischenprodukt XXV in eine Verbindung der Formel XXVI im Verfahrensschritt o) übergeführt werden. Zunächst wird die Verbindung der Formel XXIV durch Umsetzung mit einem Triarylphosphinhydrohalogenid, wie im Zusammenhang mit der Reaktion d) beschrieben, in das Phosphoniumsalz XXV übergeführt. Die Verbindung der Formel XXV wird im Reaktionsschritt p) durch Umsetzung mit dem Aldehyd der Formel VII in eine Verbindung der Formel XXVI übergeführt, vergleiche hier zu Reaktionsschema 1. Die Reaktion von Stufe p) wird in einer Wittig-Reaktion in der im Reaktionsschritt c) beschriebenen Weise durchgeführt. Die Verbindung der Formel XXVI, worin $R_9$ Niederalkyl ist, kann gewünschtenfalls durch konventionelle basische Hydrolyse in die freie Säure übergeführt werden. Andererseits kann die Verbindung der Formel XXVI, in der $R_4$ eine verätherte Hydroxygruppe enthält, durch Säurehydrolyse in den freien Alkohol übergeführt werden. Die Ätherhydrolyse einer Verbindung der Formel XXVI kann entweder vor oder nach der Säurehydrolyse durchgeführt werden. Andererseits kann die Verbindung der Formel X, in der $R_4$ eine verätherte Hydroxygruppe enthält, in der gleichen Weise hydrolysiert werden wie eine Verbindung der Formel XXVI.

Die Verbindung der Formel XXIV kann auch in das tertiäre Amin der Formel XXXI übergeführt werden. Hierfür wird die Verbindung der Formel XXIV im Reaktionsschritt q) mit dem Säurehalogenid der Formel XIX-A in der im Zusammenhang mit der Umsetzung der Verbindung XXII zu einer Verbindung der Formel XXIII beschriebenen Weise zu einer Verbindung der Formel XXVII umgesetzt.

Die Verbindung der Formel XXVII wird im Reaktionsschritt r) zu einer Verbindung der Formel XXIX durch Umsetzung mit einem Lithiumaluminiumhydrid-Reduktionsmittel umgesetzt wie im Zusammenhang mit Stufe n) beschrieben.

In der nächsten Verfahrensstufe s) wird eine Verbindung der Formel XXIX durch Umsetzung mit einem Triarylphosphinhydrohalogenid in eine Verbindung der Formel XXX übergeführt. Diese Reaktion wird in der gleichen Weise ausgeführt wie im Zusammenhang mit Stufe b) beschrieben. Die Verbindung der Formel XXX wird in Reaktionsschritt t) durch Umsetzung mit einer Verbindung der Formel VII (Formelschema 1) in eine Verbindung der Formel XXXI übergeführt. Bei dieser Reaktion wird eine Wittig-Reaktion durchgeführt. Sie kann in gleicher Weise wie im Zusammenhang mit der Reaktion c) beschrieben durchgeführt werden. Die Verbindung der Formel XXXI, in der $R_9'$ Niederalkyl ist, kann gewünschtenfalls durch basische Hydrolyse in eine entsprechende Verbindung mit einer freien Säuregruppe übergeführt werden. Andererseits kann eine in $R_4$ enthaltene geschützte Hydroxygruppe durch konventionelle Ätherhydrolyse in eine entsprechende Verbindung, in der $R_4$ eine freie Hydroxygruppe enthält, übergeführt werden. Diese Ätherhydrolyse kann vor oder nach der Hydrolyse der Estergruppe $COOR_9$ durchgeführt werden.

Falls $R_2$ im Formelschema 2 Hydroxy ist, wird diese Hydroxygruppe vorzugsweise in Form einer Estergruppe geschützt. Die Esterschutzgruppe kann nach der Bildung des Wittig-Salzes der Formel XXX entfernt werden.

Die Verbindungen der Formel I, in denen X -CH-(R$_{10}$) ist, werden aus Verbindungen der Formel

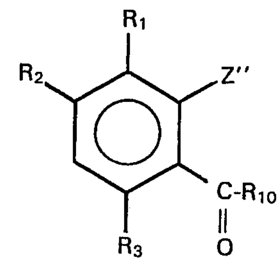

XXXV

worin Z'' Brom oder Jod; $R_{10}$ Wasserstoff oder Niederalkyl ist und $R_1$, $R_2$ und $R_3$ die obige Bedeutung haben,
gemäss Reaktionsschema 3 hergestellt.

Im Reaktionsschema 3 haben die Substituenten $R_1$, $R_2$, $R_3$, $R_4$, $R_7$, $R_8$, $R_9'$, $R_{10}$, $R_{13}$, Y Z' und Z'' die obige Bedeutung. Eine Verbindung der Formel XXXV wird im Verfahrenschritt u) durch Umsetzung mit einer Verbindung der Formel XXXIV zu einer Verbindung der Formel XXXVI umgesetzt. Diese Reaktion wird als Wittig-Reaktion durchgeführt. In der Verbindung der Formel XXXVI kann eine in $R_{13}$ enthaltene Hydroxygruppe durch Bildung einer der vorstehend erwähnten konventionellen Äthergruppen geschützt werden. Andererseits wurde gefunden, dass eine freie Hydroxygruppe nicht geschützt zu werden braucht und bei den Reaktionen in der Überführung der Verbindung der Formel XXXVI in eine Verbindung der Formel XXXXI nicht angegriffen wird. Zur Erzielung bester Ausbeuten ist es jedoch im allgemeinen bevorzugt, diese Hydroxygruppe durch eine hydrolysierbare Äthergruppe zu schützen.

Der Reaktionsschritt u) wird mittels einer Wittig-Reaktion zwischen einer Verbindung der Formel XXXV und einer Verbindung der Formel XXXIV unter Anwendung der gleichen Reaktionsbedingungen wie im Zusammenhang mit Reaktionsschritt c) beschrieben durchgeführt.

Die Verbindung der Formel XXXVI kann in eine Verbindung der Formel XXXVII im Reaktionsschritt v) durch Hydrierung übergeführt werden. Jede konventionelle Hydriermethode kann hierfür angewandt werden. Beispielsweise kann die Verbindung der Formel XXXVI mit Wasserstoffgas in Gegenwart eines Katalysators in einem inerten organischen Lösungsmittel behandelt werden. Bevorzugte Katalysatoren sind Palladiumkatalysatoren. Für die Reaktion können die konventionellen inerten organischen Lösungsmittel und die konventionellen Bedingungen für katalytische Hydrierung angewandt werden.

Im nächsten Reaktionsschritt w) wird die Verbindung der Formel XXXVII durch Behandlung mit Formaldehyd oder einer Formaldehyd freisetzenden Verbindung in eine Verbindung der Formel XXXIX übergeführt. Bei dieser Reaktion wird die Verbindung der Formel XXXVII zuerst mit einem Alkalimetallalkyl z.B. n-Butyllithium metalliert. Im allgemeinen wird diese Reaktion in einem inerten organischen Lösungsmittel z.B. einem Äther durchgeführt. Unter den bevorzugten Lösungsmittel sind Diäthyläther und Tetrahydrofuran. Bei dieser Reaktion sind Temperatur und Druck nicht kritisch. Die Reaktion kann bei Raumtemperatur und Atmosphärendruck ausgeführt werden. Gewünschtenfalls können höhere oder niedere Temperaturen angewandt werden. Nach Behandlung der Verbindung der Formel XXXVIII mit einem Alkalimetallalkyl wird dem Reaktionsmedium Formaldehyd oder eine Formaldehyd freisetzende Verbindung zugegeben. Als solche kann z.B. Paraformaldehyd angewandt werden. Die Reaktion wird im gleichen Reaktionsmedium und unter Anwendung der gleichen Bedingungen wie bei der Metallierung einer Verbindung der Formel XXXVIII durchgeführt.

Die Verbindung der Formel XXXIX wird dann im Reaktionsschritt x) durch Behandlung mit einem Triarylphosphinhydrohalogenid in eine Verbindung der Formel XXXX übergeführt. Diese Reaktion wird in der gleichen Weise wie im Zusammenhang mit Reaktionsschritt b) beschrieben durchgeführt. Die Verbindung der Formel XXXX wird im Reaktionsschritt y) durch Behandlung mit einer Verbindung der Formel VII in eine Verbindung der Formel XXXXI übergeführt (vgl. Reaktionsschema 1). Diese Reaktion y) wird als Wittig-Reaktion unter den im Zusammenhang mit der Reaktion c) beschriebenen Bedingungen durchgeführt. Die Verbindung der Formel XXXXI kann in eine entsprechende Verbindung übergeführt werden, die eine freie Carboxylgruppe anstelle der Gruppe $R_9'$ enthält. Das wird durch konventionelle Esterhydrolyse in der früher beschriebenen Weise bewerkstelligt. Wenn $R_4$ eine geschützte Hydroxygruppe enthält, kann diese Ätherschutzgruppe hydrolysiert werden durch Behandlung mit einer wässrigen anorganischen Säure. Die Hydrolyse kann entweder vor oder nach der Hydrolyse der Estergruppe in der Verbindung der Formel XXXXI durchgeführt werden.

Zur Herstellung von Verbindungen der Formel I in denen X $-C(R_{10})=CH$ darstellt, wird die Verbindung der Formel XXXV gemäss Reaktionsschema 3 und Verfahrensschritt u) mit einer Verbindung der Formel XXXIV, worin $R_{13}$ $R_4$ ist, zu einer Verbindung der Formel XXXVI, worin $R_{13}$ $R_4$ ist, umgesetzt. Die letztgenannte Verbindung wird dann der gleichen Reaktionsfolge wie die Verbindung der Formel XXXVII unterworfen, d.h. den Reaktionsschritten w), x) und y) wobei eine Verbindung der Formel X erhalten wird, in der X $-C(R_{10})=CH-$ darstellt.

Wenn in einer Verbindung der Formel XXXV im Reaktionsschema 3 $R_2$ eine Hydroxygruppe darstellt, wird diese Gruppe vorzugsweise durch Veresterung mit einer Niederalkancarbonsäure geschützt. Die Esterschutzgruppe kann nach Bildung des Wittig-Salzes der Formel XXXX gespalten werden.

Verbindungen der Formel I, in denen X $-CH(R_{10})O-$ darstellt, können aus Verbindungen der Formel

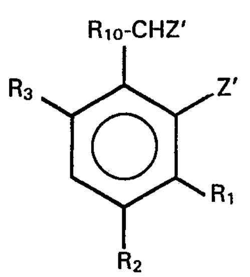

L

worin $R_1$, $R_2$, $R_3$ und Z' die obige Bedeutung haben, nach dem Reaktionsschema 4 hergestellt werden. In den dort angegebenen Formeln haben $R_1$, $R_2$, $R_3$, $R_7$, $R_8$, $R_{10}$, $R_9'$, Y und Z'' die obige Bedeutung, $R_{15}$ ist ein Rest $R_4$, in dem die darin enthaltene Hydroxylgruppe in Form einer hydrolysierbaren Äthergruppe wie Tetrahydropyranyl oder die früher erwähnten Äthergruppen geschützt ist.

Die Verbindung der Formel L wird durch Reaktion mit einem Alkalimetallalkoxid der Formel L-A in eine Verbindung der Formel LI übergeführt. Die Reaktion wird nach üblichen Verfahrensbedingungen für die Umsetzung von Alkalimetallalkoxiden mit einem Halogenid durchgeführt.

Die Verbindung der Formel LI wird in eine Verbindung der Formel LII übergeführt, indem man zunächst die Verbindung der Formel LI mit einem Lithiumalkyl wie n-Butyllithium metalliert. Die metallierte Verbindung der Formel LI wird danach mit Formaldehyd oder einer Verbindung, die Formaldehyd abgibt umgesetzt. Bei der Umwandlung der Verbindung der Formel LI in eine Verbindung der Formel LII werden die gleichen Reaktionsbedingungen angewandt wie im Zusammenhang mit dem Reaktionsschritt w) beschrieben. Die Verbindung der Formel LII wird in das Phosphoniumsalz der Formel LIII durch Behandlung mit einem Triarylphosphinhydrohalogenid in der in Reaktionsstufe b) beschriebenen Weise übergeführt. Das Phosphoniumsalz der Formel LIII wird in einer Wittig-Reaktion mit der Verbindung der Formel VII (Reaktionsschema 1) zu einer Verbindung der Formel LIV umgesetzt. Diese Reaktion wird in der gleichen Weise wie im Zusammenhang mit Reaktionsschritt c) beschrieben durchgeführt.

Wenn $R_{15}$ in der Verbindung der Formel LIV eine geschützte Hydroxygruppe enthält, kann diese durch konventionelle Hydrolysemethoden zur freien Hydroxygruppe hydrolysiert werden. Die üblichen Hydrolysemethoden für Ätherschutzgruppen greifen die andere in der Verbindung der Formel LIV enthaltene Äthergruppe nicht an. Die Verbindung der Formel LIV kann durch konventionelle Esterhydrolyse in die freie Säure übergeführt werden.

Wenn $R_2$ in den Verbindungen der Formeln L, LI, LII und LIII Hydroxy ist, wird die Hydroxygruppe vorzugsweise durch eine hydrolysierbare Estergruppe wie Niederalkanoyloxy geschützt. Die hydrolysierbare Estergruppe kann nach Bildung des Wittig-Salzes der Formel LIII abgespalten werden.

Die Doppelbindungen in der Verbindung der Formel I in den Stellungen 2-3, 4-5, 6-7 und 8-9 können entweder cis- oder trans-Konfiguration aufweisen. Andererseits können die Verbindungen als Gemisch der verschiedenen cis- und trans-Isomeren vorliegen. In der Verbindung der Formel VII können die darin enthaltenen Doppelbindungen entweder cis- oder trans-Konfiguration haben jenachdem welche Stereokonfiguration der Doppelbindungen in den Verbindungen der Formel I erwünscht ist. Die bei der Herstellung der Verbindungen der Formel I in den Stufen c), e), i) usw. durchgeführte Wittig-Reaktion liefert ein Gemisch von 8-9 cis/trans-Isomeren. Diese cis/trans-Isomeren können durch herkömmliche Mittel wie fraktionierte Kristallisation getrennt werden.

Wenn die Verbindungen der Formel I eine 2-3 trans-Doppelbindung haben, kann dieses Isomer in die entsprechende cis-Doppelbindung mittels an sich bekannter Isomerisationsmethoden umgewandelt werden. Beispielsweise kann eine Verbindung der Formel I mit Jod in einem inerten organischen Lösungsmittel behandelt werden. Isomerisierung mit Jod liefert Verbindungen der Formel I mit 2-3 cis-Doppelbindungen.

Die Formel I umfasst alle geometrischen Isomeren einschliesslich Mischungen dieser geometrischen Isomeren.

Verbindungen der Formel XI, worin $R_1$ Fluor ist, sind neu und können aus Verbindung der Formel

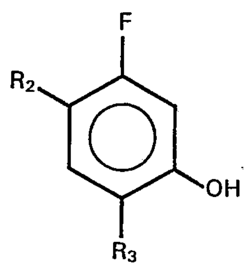

LV

worin $R_2$ und $R_3$ die obige Bedeutung haben, nach dem Reaktionsschema 5 hergestellt werden, wobei $R_2$, $R_3$ und $R_4$ die obige Bedeutung haben.

Im Reaktionsschema 5 wird die Verbindung LV durch Umsetzung mit einem Allylbromid alkyliert. Eine Hydroxygruppe $R_2$ wird hierbei durch Veresterung geschützt, d.h. $R_2$ in der Verbindung der Formel LV ist eine geschützte Hydroxygruppe. Für die Umsetzung einer Verbindung der Formel LV zu einer Verbindung der Formel LVI können die für Alkylierungen einer Hydroxygruppen mit einem Allylbromid bekannten Methoden Anwendung finden. Die Verbindung der Formel LVI wird durch Erwärmen auf eine Temperatur von 190-230°C zu einer Verbindung der Formel LVII umgelagert. Diese Umlagerung kann in Gegenwart eines hochsiedenden Kohlenwasserstoffes oder ohne Verwendung irgendeines Lösungsmittels stattfinden. Wenn $R_3$ Wasserstoff ist, wird die Verbindung der Formel LVII als Gemisch des Isomeren mit der Verbindung der Formel LVII erhalten in der die Allylgruppe para zum Fluorsubstituenten am Phenylring steht. Dieses Isomer kann abgetrennt werden oder in den nachfolgenden Reaktionen mitgeführt und aus dem Reaktionsgemisch auf einer späteren Stufe abgetrennt werden.

Die Verbindung der Formel LVII wird danach durch Umsetzung mit einer Verbindung der Formel V (Reaktionsschema 1) gemäss Reaktionsschritt d) zu einer Verbindung der Formel LVIII umgesetzt. Im nächsten Reaktionsschritt wird die Verbindung der Formel LVIII durch Isomerisierung mit einer starken Base wie einem Alkalimetallalkoxid in Gegenwart eines inerten organischen Lösungsmittels, vorzugsweise Kalium-t-butoxid in Dimethylsulfoxid, zu einer Verbindung der Formel LIX isomerisiert. Die Verbindung der Formel LIX wird in eine Verbindung der Formel LX durch Behandlung mit Ozon übergeführt. Hierbei werden Temperaturen von −70 bis −20°C angewandt. Weiterhin arbeitet man bei dieser Temperatur in einem inerten organischen Lösungsmittel. Jedes konventionelle inerte organische Lösungsmittel kann Verwendung finden, bevorzugt sind halogenierte Kohlenwasserstoffe wie Methylenchlorid.

Eine Verbindung der Formel LX ist eine Verbindung der Formel XI mit $R_1$ = Fluor. Diese Verbindung kann in eine Verbindung der Formel X gemäss dem Reaktionsschema 1 übergeführt werden.

Wenn in einer Verbindung der Formel III $R_2$ Hydroxy ist, liegen 2 Hydroxygruppen vor. Es ist daher im allgemeinen vorzuziehen, die Verbindung der Formel XI, in der $R_2$ eine geschützte Hydroxygruppe darstellt, aus einer Verbindung der Formel LXI (Reaktionsschema 6) herzustellen. Auf diese Weise können Verbindungen der Formel I hergestellt werden, in denen X -O- und $R_2$ Hydroxy ist und $R_3$ und $R_4$ die obige Bedeutung haben. Im Reaktionsschema 6 sind $R_1$ und $R_{15}$ zusammen mit dem daran gebundenen Sauerstoffatom durch eine konventionelle hydrolysierbare Schutzgruppe, vorzugsweise Niederalkanoyl, geschütztes Hydroxy.

Im Reaktionsschema 6 wird die Verbindung der Formel LXI in eine Verbindung der Formel LXII übergeführt, wobei die gleichen Reaktionsbedingungen wie im Zusammenhang mit der Umwandlung einer Verbindung der Formel LV in eine Verbindung der Formel LVI (Reaktionsschema 5) beschrieben Anwendung finden. Die Verbindung der Formel LXII wird dann nach dem gleichen Verfahren wie im Zusammenhang mit der Umwandlung der Verbindung LVI in eine Verbindung der Formel LVII beschrieben in eine Verbindung der Formel LXIII übergeführt. In den nächsten Stufen wird die Verbindung der Formel LXIII in eine Verbindung der Formel LXIV nach Verfahren, die im Schritt g') in Reaktionsschema 5 beschrieben sind, und danach zu einer Verbindung der Formel LXV nach dem im Zusammenhang mit Stufe h') in Schema 5 beschriebenen Verfahren übergeführt. Die Umwandlung der Brombenzolverbindung der Formel LXV in das Phenol der Formel LXVI wird nach an sich bekannten Verfahren (Kidwell and Darling, Tetrahedron Letters, (1966) Seiten 531-535) durchgeführt.

Im nächsten Schritt der Herstellung des Zwischenproduktes der Formel XI, in dem $R_2$ eine geschützte Hydroxygruppe ist, d.h. der Verbindung XI-A wird die Hydroxygruppe in der Verbindung der Formel LXVI in eine konventionell hydrolysierbare Estergruppe übergeführt, wobei man die Formel LXVII erhält in der $R_{15}$ zusammen mit dem Sauerstoffatom, an das sie gebunden ist, eine hydrolysierbare Estergruppe darstellt. Für diese Veresterung können konventionelle Methoden der Veresterung von Hydroxygruppen mit organischen Säuren wie Niederalkansäuren mit 1-7 C-Atomen angewandt werden. Die Verbindung der Formel XI-A wird aus der Verbindung der Formel LXVII in der gleichen Weise hergestellt wie vorstehend inbezug auf die Umwandlung der Verbindung der Formel LIX in L (Schema 5) beschrieben.

Bei der Umwandlung der Verbindung der Formel XI-A in einer Verbindung der Formel XVII (Reaktionsschema 1) ist es im allgemeinen vorzuziehen, den

Estersubstituenten $R_2$ nach der Bildung des Wittig-Salzes der Formel XIII oder XVI zu hydrolysieren.

In einer anderen Ausführungsform wird eine Verbindung der Formel XI, in der $R_1$ Trifluormethyl ist (die Verbindung der Formel XI-B), gemäss Reaktionsschema 7 aus einer Verbindung der Formel LXX hergestellt. Im ersten Schritt dieser Reaktion wird die Verbindung der Formel LXX in eine Verbindung der Formel LXXI wie im Zusammenhang mit der Reaktion d) (Umsetzung der Verbindung VIII mit einer Verbindung der Formel V zu einer Verbindung der Formel X) beschrieben übergeführt. Wenn hierbei $R_2$ Hydroxy ist, verläuft die Alkylierung an der zur Trifluormethylgruppe ortho-ständigen Hydroxygruppe sehr langsam. Infolgedessen muss diese Gruppe nicht notwendigerweise geschützt werden, da die Alkylierung vorzugsweise mit der meta-ständigen Hydroxygruppe erfolgt. Mischungen alkylierter Produkte, die bei dieser Reaktion erhalten werden, können durch herkömmliche Verfahren getrennt werden. Die Verbindung der Formel LXXI wird in eine Verbindung der Formel XII-B nach herkömmlichen Verfahren zur Formylierung des Benzolringes, z.B. durch Behandlung mit Lithiumalkyl und Dimethylformamid, umgewandelt.

Die nachstehenden Beispiele erläutern die Erfindung weiter. In den Beispielen ist der verwendete Äther Diäthylätheräther, die Lösungsmittel wurden unter vermindertem Druck entfernt.

*Beispiel 1*

Ein Gemisch von 110 g 2-Hydroxybenzaldehyd, 180 g 1-Bromnonan, 800 ml Dimethylformamid und wasserfreies Kaliumcarbonat wurden 14 Stunden auf 80° erwärmt. Danach wurden Hexan und Wasser zugegeben. Die Hexanphase wurde konzentriert und der Rückstand destilliert. Man erhielt 210 g 2-Nonyloxybenzaldehyd, Siedepunkt 121°/40 Pa. Eine Lösung von 100 g 2-Nonyloxybenzaldehyd in 1000 ml Äthanol wurde mit 6 g Natriumborhydrid bei 10° versetzt. Das Gemisch wurde 15-20 Minuten bei Raumtemperatur gerührt und der 2-Nonyloxybenzylalkohol mit Hexan extrahiert. Entfernung des Lösungsmittels unter vermindertem Druck lieferte 98 g rohen Z-Nonyloxybenzylalkohol, der zu einem Gemisch von 144 g Triphenylphosphinhydrobromid in 500 ml Acetonitril gegeben wurde. Die erhaltene Lösung wurde 14 Stunden zum Rückfluss erhitzt. Entfernung des Lösungsmittels unter vermindertem Druck und Kristallisation des Rückstandes aus Tetrahydrofuran/Äthyläther lieferte reines [[2-(Nonyloxy)phenyl]-methyl]triphenylphosphoniumbromid. Ausbeute 208 g.

*Beispiel 2*

Behandlung von 2-Hydroxybenzylalkohol mit Triphenylphosphinhydrobromid in Acetonitril nach dem in Beispiel 1 beschriebenen Verfahren lieferte [(2-Hydroxyphenyl)-methyl]triphenylphosphoniumbromid.

*Beispiel 3*

Eine Lösung von einem Mol [(2-Hydroxyphenyl)-methyl]triphenylphosphoniumbromid in Tetrahydrofuran wurde durch Behandlung mit 2,1 Mol-Äquivalent n-Butyllithium in Hexan bei −35° in das Ylid übergeführt, danach mit einem Mol 7-Formyl-3-methyl-2,4,6-octatriensäureäthylester versetzt und 15 Minuten bei −70° gerührt. Isolierung der organischen Stoffe mit Hexan/Äthylacetat (4:1 Volumenteile) und verdünnter Minerasäure (2M wässrige HCl) lieferte reinen all(E)-9-(2-Hydroxyphenyl)-3,7-dimethyl-2,4,6,8-nona-tetraensäureäthylester in 90% Ausbeute nach Chromatographie und Kristallisation aus Dichlormethan/Hexan.

*Beispiel 4*

Ein Gemisch von 0,5 Mol 2-Hydroxybenzaldehyd, 0,6 Mol (7-Carboxy-2,6-dimethyl-2,4,6-heptatrien-1-yl)triphenylphosphoniumbromid in 750 ml 1,2-Epoxybutan wurde 30 Minuten zum Rückfluss erhitzt, danach gekühlt, in eine Äther/Hexangemisch (1:1 Volumenteile) gegossen, filtriert und konzentriert. Der Rückstand wurde aus Hexan/Äther kristallisiert und lieferte all(E)-9-(2-Hydroxyphenyl)-3,7-dimethyl-2,4-6,8-nonatetraensäureäthylester in 38% Ausbeute, Schmelzpunkt 143-145°.

*Beispiel 5*

Eine Lösung von 150 g [[2-(Nonyloxy)phenyl]methyl]triphenylphosphoniumbromid in 1100 ml Tetrahydrofuran wurde auf −50° gekühlt. Dabei wurde eine Suspension des festen Salzes erhalten. Zu diesem Gemisch wurde eine Lösung von 180 ml 1,6 molares n-Butyllithium in Hexan gegeben. Das Gemisch wurde dann weitere 15 Minuten bei −40° gerührt, danach auf −70° gekühlt und mit 65 g 7-Formyl-3-methyl-2,4,6-octatriensäureäthylester in 250 ml Tetrahydrofuran versetzt. Zusatz von Hexan und wässrigem Methanol (40%) und nachfolgende Konzentration des Hexanextraktes lieferte 64 g (58%) all(E)-9-[2-(Nonyloxy)phenyl]-3,7-dimethyl-2,4,6,8-nonatetraensäureäthylester, Schmelzpunkt 52-53°. Dieser Ester wurde wie folgt hydrolysiert: 70 g Ester wurden in 1000 ml Äthanol gelöst. Die Lösung wurde mit einer Lösung von 80 g Kaliumhydroxid in 400 ml Wasser versetzt und 1 Stunde zum Rückfluss erhitzt. Danach wurden Wasser und wässrige Mineralsäure zugesetzt und die Feststoffe mit Chloroform aufgenommen. Konzentration dieses organischen Extraktes und Kristallisation des Rückstandes aus Äthylacetat/Hexan lieferte all(E)-9-[2-(Nonyloxy)phenyl]-3,7-dimethyl-2,4,6,8-nonatetraensäure vom Schmelzpunkt 102-103°, Ausbeute 38 g.

*Beispiel 6*

Ein Gemisch von 24 g Natriumhydrid (50 Gew.-% in Mineralöl) und 1000 ml Dimethylformamid wurden bei 10° mit 0,4 Mol-Äquivalenten all(E)-9-(2-Hydroxyphenyl)-3,7-dimethyl-2,4,6,8-nonatetraensäureäthylester versetzt. Das erhaltene Gemisch wurde bei Raumtemperatur gerührt, bis die Wasserstoffentwicklung aufgehört hatte und das Natriumsalz gebildet worden war. Eine Lösung von 0,5 Mol-Äquivalenten 1-Nonyltosylat in 200 ml Dimethylformamid wurde dann zu dieser Salzlösung gegeben und das Reaktionsgemisch wurde 14 Stunden bei 45° gerührt. Danach wurden sorgfältig Hexan und Wasser zugesetzt, der Hexanextrakt wurde konzentriert und der Rückstand durch Chromatographie an Silicagel gereinigt. Kristallisation aus Hexan lieferte dann reinen (All-E)-9-[2-Nonyloxy)phenyl]-3,7-dimethyl-2,4,6,8-nonatetraensäureäthylester. Hydrolyse dieses Esters wie in Beispiel 5 lieferte (All-E)-9-[2-(Nonyloxy)phenyl]-3,7-dimethyl-2,4,6,8-nonatetraensäure.

*Beispiel 7*

2-Hydroxybenzaldehyd wurde mit 2,2-Dimethyl-1-jodoctan zu 2-(2,2-Dimethyloctyloxy)benzaldehyd kondensiert, der zu 2-(2,2-Dimethyloctyloxy)-benzylalkohol reduziert und dann gemäss Beispiel 1 in [[2-(2,2-Dimethyloctyloxy)phenyl]methyl]-triphenylphosphoniumbromid umgewandelt wurde. Kondensation dieses Phosphoniumbromids mit 7-Formyl-3-methyl-2,4,6-octatriensäureäthylester gemäss Beispiel 3 und anschliessende Hydrolyse gemäss Beispiel 5 lieferte (All-E)-3,7-Dimethyl-9-[2-[(2,2-dimethyl-octyl)oxy]phenyl]-2,4,6,8-nonatetraensäure vom Schmelzpunkt 113-117° (aus Dichlormethan/Hexan).

*Beispiel 8*

Aus Octanol und n-Butyllithium hergestelltes Lithiumoctanat wurde in einem Gemisch von Tetrahydrofuran/Hexan und Dimethylformamid mit 2-Brombenzylbromid zu 2(Octyloxy)-methylbrombenzol kondensiert. Dieses Material wurde in Äther/Hexan mit n-Butyllithium behandelt und danach mit Paraformaldehyd, wobei 2-(Octyloxy)methylbenzylalkohol erhalten wurde. Dieses Material wurde dann mit Triphenylphosphoniumbromid zu [[2-[(Octyloxy)-methyl]phenyl]methyl]triphenylphosphoniumbromid umgesetzt. Kondensation dieses Materials mit 7-Formyl-3-methyl-2,4,6-octatriensäureäthylester wie in Beispiel 3 und anschliessende Hydrolyse wie in Beispiel 5 lieferte (All-E)-3,7-Dimethyl-9-[2-[(octyloxy)-methyl]phenyl]-2,4,6,8-nonatetraensäure vom Schmelzpunkt 120-121° (aus Dichlormethan/Hexan).

*Beispiel 9*

2-Chlor-6-hydroxy-benzaldehyd wurde wie in Beispiel 1 mit 1-Bromnonan zu 2-Chlor-6-nonyloxy-benzaldehyd alkyliert. Reduktion mit Natriumborhydrid wie in Beispiel 1 lieferte 2-Chlor-6-nonylbenzylalkohol der durch Behandlung mit Triphenylphosphinhydrobromid in Acetonitril wie in Beispiel 1 [[2-Chlor-6-nonyloxy]phenyl]methyl]triphenylphosphoniumbromid lieferte. Kondensation mit 7-Formyl-3-methyl-2,4,6-octatriensäureäthylester wie in Beispiel 3 lieferte (All-E)-9-[2-Chlor-6-(nonyloxy)phenyl]-3,7-dimethyl-2,4,6,8-nonatetraensäureäthylester. Dieser Ester wurde wie in Beispiel 5 hydrolysiert und lieferte (All-E)-9-[2-Chlor-6-(nonyloxy)phenyl]-3,7-dimethyl-2,4,6,8-nonatetraensäure vom Schmelzpunkt 129-131° (aus Äthylacetat/Hexan).

*Beispiel 10*

5-Methoxy-2-hydroxybenzaldehyd wurde mit Nonylbromid gemäss Beispiel 1 alkyliert und mit Natriumborhydrid reduziert zu 5-Methoxy-2-nonyloxy-benzylalkohol, der durch Behandlung mit Triphenylphosphinhydrobromid das [(5-Methoxy-2-nonyloxyphenyl)methyl]triphenylphosphoniumbromid lieferte. Kondensation dieses Materials mit 7-Formyl-3-methyl-2,4,6-octatriensäureäthylester gemäss Beispiel 3 und anschliessende Hydrolyse gemäss Beispiel 5 lieferte (All-E)-9-(5-Methoxy-2-nonyloxyphenyl)-3,7-dimethyl-2,4,6,8-nonatetraensäure vom Schmelzpunkt 125-126° (aus Methanol).

*Beispiel 11*

Das gemäss Beispiel 6 hergestellte Natriumsalz von all(E)-9-(2-Hydroxyphenyl)-3,7-dimethyl-2,4,6,8-nonatetraensäureäthylester in Dimethylformamid wurde wie in Beispiel 6 mit 1,8-Dihydroxyoctanmonotosylat behandelt und lieferte all(E)-9-[2-[8-Hydroxyoctyl)oxy]phenyl]-3,7-diäthyl-2,4,6,8-nonatetraensäureäthylester nach Chromatographie über Silicagel. Hydrolyse wie in Beispiel 5 lieferte all(E)-9-[2-[(8-Hydroxy-octyl)oxy]phenyl]-3,7-dimethylphenyl]-3,7-dimethyl-2,4,6,8-nonatetraensäure vom Schmelzpunkt 122-123° (aus Äthylacetat).

*Beispiel 12*

62 g 2-Nonyloxy)benzaldehyd in 500 ml Aceton wurden mit 100 ml 1M wässrigem Natriumhydroxid 18 Stunden bei Raumtemperatur behandelt. Danach wurden Kochsalzlösung und Äthylacetat/Hexan (1:1 Volumen) zugesetzt. Einengen der organischen Phase und anschliessende Kristallisation aus Hexan lieferte 4-(2-Nonyloxyphenyl)-3-buten-2-on in einer Ausbeute von 53 g.

Eine Lösung von 58 g 4-(2-Nonyloxyphenyl)-3-buten-2-on in 200 ml Tetrahydrofuran wurde zu einer Lösung von 200 ml 1,6M Vinylmagnesiumbromid in Tetrahydrofuran, die mit weiterem Tetrahydrofuran auf 1 l verdünnt wurde bei −30° gegeben. Danach wurde das Gemisch 30 Minuten bei 0° gerührt, mit 100 ml wässriger Ammoniumchloridlösung und 2 l Äther versetzt und von den Feststoffen abdestilliert. Einengen des organischen Extraktes und Reinigung durch Chromatographie an Silicagel lieferte 40 g (E)-5-(2-Nonyloxyphenyl)-3-hydroxy-3-methyl-1,4-pentadien als Öl.

Eine Lösung von 66 g (E)-5-(2-Nonyloxyphenyl)-3-hydroxy-3-methyl-1,4-pentadien in 250 ml Acetonitril wurde bei 10° zu einer Aufschlämmung von 66 g Triphenylphosphoniumhydrobromid in 300 ml Acetonitril gegeben. Nach Erwärmen auf Raumtemperatur wurde das Gemisch 2 Stunden bei dieser Temperatur gerührt bis Lösung eingetreten war. Diese Lösung wurde dann mit 2 × 250 ml Hexan extrahiert und die Acetonitrilschicht konzentriert (ca. 400 ml) und auf −10° gekühlt. Die Feststoffe wurden abfiltriert, mit Acetonitril und Hexan gewaschen und getrocknet. Man erhielt 21 g reines all(E)-[5-(2-Nonyloxyphenyl)-3-methyl-2,4-pentadienyl]triphenylphosphoniumbromid.

*Beispiel 13*

Ausgehend von all(E)-[5-(2-Nonyloxyphenyl)-3-methyl-2,4-pentadienyl]triphenylphosphoniumbromid wurde mittels des Verfahrens von Beispiel 5 das Ylid mit 3-Formyl-2-butensäureäthylester zu all(E)-9-(2-nonyloxyphenyl)-3,7-dimethyl-2,4,6,8-nonatetraensäure umgesetzt.

*Beispiel 14*

Eine Lösung von 10 g all(E)-9-[2-(Nonyloxy)phenyl]-3,7-dimethyl-2,4,6,8-nonatetraensäureäthylester in 200 ml Hexan, die 0,5 g Jod enthielten, wurde 30 Minuten bei Raumtemperatur gerührt. Das Hexan wurde mit wässriger Natriumthiosulfatlösung (10 Gew.-%) jodfrei gewaschen, getrocknet und eingeengt, wobei ein Gemisch von Doppelbindungsisomeren erhalten wurde. Trennung durch Chromatographie an Silicagel lieferte 1,5 g reinen (Z,E,E,E)-9-[2-(Nonyloxy)phenyl]-3,7-dimethyl-2,4,6,8-nonatetraensäureäthylester. Hydrolyse mit wässrig-äthanolischer Kaliumhydroxidlösung unter Rückfluss lieferte reine (Z,E,E,E)-9-[2-(Nonyloxy)phenyl]-3,7-dimethyl-2,4,6,8-nonatetraensäuresäure, Schmelzpunkt 135-136°.

*Beispiel 15*

Die Mutterlauge bei der Kristallisation von all(E)-9-[2-(Nonyloxy)phenyl]-3,7-dimethyl-2,4,6,8-nonatetraensäureäthylester aus Beispiel 5 stellte ein Gemisch verschiedener Isomere dar. Reinigung durch Chromatographie lieferte 80% reinen Äthylester, der nach Hydrolyse wie in Beispiel 5 reine (E,E,E,Z)-9-[2(Nonyloxy)phenyl]-3,7-dimethyl-2,4,-6,8-nonatetraensäure vom Schmelzpunkt 105-109° lieferte.

*Beispiel 16*

0,1 Mol Nonylmethyltriphenylphosphoniumbromid in 200 ml Tetrahydrofuran wurde bei −10° mit 0,1 Mol-Äquivalent n-Butyllithium (1,6 Mol in Hexan) in das Ylid übergeführt.

Danach wurden 0,09 Mol 2-Brombenzaldehyd in 25 ml Tetrahydroduran zugesetzt und das Gemisch 30 Minuten bei 0° gerührt und mit Hexan und wässrigem Methanol (40:60) versetzt. Der Hexanextrakt wurde eingeengt und der Rückstand destilliert. Man erhielt 2-(1-Decenyl)-1-brombenzol in 90% Ausbeute.

Dieses Material wurde in Hexan gelöst und in Gegenwart von Palladium/Kohle bei Raumtemperatur und Raumdruck hydriert, bis die olefinische Bindung gesättigt war. Die Feststoffe wurden abfiltriert. Entfernung des Hexans und Destillation des Rückstandes lieferten 2-Decyl-1-brombenzol, Siedepunkt 120°/0,133 Pa in einer Ausbeute von 80%.

*Beispiel 17*

Eine Lösung von 0,1 Mol 2-Decyl-1-brombenzol in 150 ml Äther wurde mit 0,11 Äquivalent n-Butyllithium (1,6M in Hexan) versetzt und das Gemisch 2 Stunden bei Raumtemperatur gerührt. Danach wurden 0,2 Mol-Äquivelent trockener Paraformaldehyd zugegeben und das Gemisch weitere 18 Stunden bei Raumtemperatur gerührt. Danach wurden Wasser und Äther zugesetzt und die ätherischen Extrakte getrocknet und konzentriert. Der Rückstand lieferte nach Chromatographie reines 2-Decyl-1-hydroxymethylbenzol in 75% Ausbeute.

*Beispiel 18*

2-Decyl-1-hydroxymethylbenzol wurde nach dem Verfahren von Beispiel 1 mit Triphenylphosphoniumhydrobromid in Acetonitril in das Phosphoniumsalz übergeführt. Dieses Salz wurde, wie vorstehend beschrieben, mit n-Butyllithium in Tetrahydrofuran umgesetzt und danach mit 7-Formyl-3-methyl-2,4,6-heptatriensäure-methylester behandelt. Reinigung des rohen Kondensationsproduktes durch Chromatographie an Silicagel und anschliessende basische Hydrolyse lieferte reine all(E)-9-(Decylphenyl)-3,7-dimethyl-2,4,6,8-nonatetraensäure vom Schmelzpunkt 107-108° (aus Hexan-Äther).

*Beispiel 19*

1 Mol 2-Aminobenzylalkohol wurde mit 2,2 Mol Octanoylchlorid in einem Gemisch von Dichlormethan-Triäthylamin bei 0° umgesetzt. Nach 30 Minuten bei 10° wurde das Gemisch mit Wasser gewaschen und der Äther abdestilliert. Der rohe Rückstand wurde in 2000 ml Tetrahydrofuran gelöst, mit 1500 ml 1N wässriger Natronlauge behandelt und 3 Stunden bei Raumtemperatur gerührt.

Zusatz von Wasser und Äther lieferte das rohe Hydroxymethyloctylamid. Reinigung durch Chromatographie lieferte reines Octylamid in 85% Ausbeute.

100 g dieses Materials wurden in 500 ml Tetrahydrofuran gelöst und zu einer Aufschwemmung von 2 Mol-Äquivalent Lithiumaliminiumhydrid in 1000 ml Tetrahydrofuran gegeben. Das Gemisch wurde dann 8 Stunden zum Rückfluss erhitzt, auf 0° gekühlt und mit 100 ml wässriger Natriumsulfatlösung versetzt.

Die Feststoffe wurden abfiltriert, die Lösungsmittel unter vermindertem Druck entfernt und der Rückstand durch Chromatographie an Silicagel gereinigt. Man erhielt 75 g reines 2-Hydroxymethyl-N-octylamin.

Dieses Material wurde in 300 ml Acetonitril, die 1,1 Mol-Äquivalente Triphenylphosphinhydrobromid enthielten, gelöst. Das Gemisch wurde 24 Stunden zum Rückfluss erhitzt und dann eingeengt. Der Rückstand wurde mit Äther behandelt und lieferte das Phosphoniumsalz als weissen Feststoff.

Dieses Material wurde 1 Stunde unter Rühren bei 0° mit 1,5 Mol-Äquivalent n-Butyllithium umgesetzt und lieferte das entsprechende Ylid. Danach wurden 1,6 Mol-Äquivalente 7-Formyl-3-methyl-2,4,6-heptatriensäureäthylester in Tetrahydrofuran zugesetzt und das Gemisch 1 Stunde bei 10° gerührt.

Zusatz von Hexan und wässrigem Methanol (2:3) und Entfernung des Hexans unter vermindertem Druck lieferte ein Rohprodukt, das nach Reinigung durch Chromatographie an Silicagel und Kristallisation aus Hexan reinen all(E)-9-(2-Octylaminophenyl)-3,7-dimethyl-2,4,6,8-nonatriensäureäthylester vom Schmelzpunkt 38-40° in einer Ausbeute von 25% lieferte.

*Beispiel 20*

Eine Lösung von 100 g 3-Fluorphenol in 1000 ml Dimethylformamid, das 165 g Kaliumcarbonat enthielt, wurde mit 115 g Allylbromid versetzt und 18 Stunden auf 80° erwärmt.

Danach wurden Wasser und Hexan zugesetzt, der Hexanextrakt wurde mit 5%iger wässriger Natriumhydroxidlösung, und gesättigter Kochsalzlösung gewaschen und konzentriert und lieferte 155 g Allyläther. 134 g dieses Materials wurden 16 Stunden auf 220° erwärmt, wobei ein Gemisch von 3-Fluor-2-(2--butenyl)phenol und 5-Fluor-2-(2-butenyl)phenol erhalten wurde. Dieses Gemisch wurde in 2000 ml Dimethylformamid mit 170 g 1-Bromnonan und 150 g Kaliumcarbonat 16 Stunden auf 80° erwärmt. Nach Verdünnen mit Wasser und Extraktion mit Hexan und Einengen wurde ein Gemisch von Produkten erhalten. Destillation lieferte ein Gemisch von 3-[(2--Fluor-6-nonyloxy)phenyl]-buten und 3-[(3-Fluor-2--nonyloxy)phenyl]-buten, Siedepunkt 120-125°/ 13,3 Pa; Ausbeute 186 g).

185 g dieses Isomerengemisches wurden in 1000 ml Dimethylsulfoxid mit 1,5 g Kalium-t-butoxid 6 Stunden bei Raumtemperatur stehengelassen. Zusatz von Wasser und Extraktion mit Hexan lieferte ein Gemisch von 1-[(2-Fluor-6-nonyloxy)-phenyl]--buten und 1-[(3-Fluor-2-nonyloxy)phenyl]-buten.

175 g dieses Isomerengemisches wurden in 2000 ml eines Gemisches von Dichlormethan und Methanol (9:1) gelöst und 8 Stunden bei -40° einem Ozonstrom ausgesetzt. Danach wurde das Reaktionsgemisch in 100 ml eines Gemisches von Wasser, Hexan und Dimethylsulfid gegossen und 1 Stunde bei Raumtemperatur gerührt.

Der Hexanextrakt wurde mit Wasser gewaschen, über Magnesiumsulfat getrocknet, mit 50 ml Dimethylsulfid versetzt und 16 Stunden bei Raumtemperatur stehengelassen.

Entfernung der Lösungsmittel lieferte 155 g eines Gemisches von 2-Fluor-6-nonyloxybenzaldehyd und 4-Fluor-2-nonyloxy-benzaldehyd.

150 g dieses Aldehydgemisches wurden in 2000 ml Äthanol mit 15 g Natriumborhydrid bei 5° versetzt und dann 30 Minuten bei Raumtemperatur gerührt. Danach wurden 1500 ml Wasser und 500 ml Kochsalzlösung zugesetzt und das Alkoholgemisch in Hexan aufgenommen. Entfernung der Lösungsmittel und Chromatographie des Rückstandes an Silicagel (5% Äthylacetat/Hexan) lieferte 76 g reinen 2-Fluor-6-nonyloxy-benzylalkohol.

*Beispiel 21*

Ein Gemisch von 19 g 2-Fluor-6-nonyloxybenzylalkohol und 26 g Triphenylphosphinhydrobromid in 250 ml Acetonitril wurde 14 Stunden zum Rückfluss erhitzt, und danach zur Trockene eingeengt. Man erhielt 42 g [[(2-Fluor-6-nonyloxy)phenyl]methyl]-triphenylphosphoniumbromid. Dieses Phosphoniumsalz wurde in 600 ml Tetrahydrofuran gelöst, und die Lösung bei -50° mit 45 ml 1,6M n-Butyllithium in Hexan vesetzt. Nach 15 Minuten Rühren bei -50° wurden 8,4 g 7-Formyl-3-methyl-2,4,6-octatriensäureäthylester zugesetzt. Das Reaktionsgemisch wurde auf Zimmertemperatur aufgewärmt und weitere 15 Minuten gerührt. Danach wurde Hexan zugesetzt und des Gemisch mit Wasser, 40%igem wässrigen Methanol gewaschen und über Magnesiumsulfat getrocknet. Einengen des Hexanextraktes und Reinigung durch Chromatographie (5% Äther-Hexan) lieferte 11 g reines trans-Isomer.

Kristallisation aus Hexan-Äthylacetat lieferte (all-E)-9-[2-Fluor-6-(nonyloxy)phenyl]-3,7-dimethyl-2,4,6,8-nonatetraensäureäthylester in einer Ausbeute von 9,5 g).

Eine Lösung von 6,5 g Ester in 150 ml Äthanol wurde mit 7 g Kaliumhydroxid in 40 ml Wasser 1 Stunde zum Rückfluss erhitzt. Das gekühlte Reaktionsgemisch wurde in kalte wässrige Salzsäure gegossen und mit Chloroform extrahiert. Entfernung des Lösungsmittels und Kristallisation aus Hexan-Äthylacetat lieferte reine (all-E)-9-[2-Fluor-6-(nonyloxy)-phenyl]-3,7-dimethyl-2,4,6,8-nonatetraensäure vom Scmelzpunkt 107-109°.

*Beispiel 22*

*Kapselformulierung:*

| *Inhaltsstoffe* | *mg/Kapsel* | | |
|---|---|---|---|
| 1. (all-E)-9-[2-nonyl-oxy)-phenyl]-3,7--dimethyl-2,4,6,8--nonatetraensäure | 15 | 30 | 60 |
| 2. Lactose | 239 | 224 | 194 |
| 3. Stärke | 30 | 30 | 30 |
| 4. Talk | 15 | 15 | 15 |
| 5. Magnesium | 1 | 1 | 1 |
| Kapselfüllgewicht | 300 mg | 300 mg | 300 mg |

*Verfahren:*

1) Mischen der Inhaltsstoffe 1-3.
2) Zusatz von Talk und Magnesiumstearat und kurzzeitiges Mischen.
3) Verkapseln.

*Beispiel 23*

Nach der Vorschrift von Beispiel 22 werden Kapseln hergestellt, die als aktiven Wirkstoff (all-E)--9-[2-Fluor-6-(nonyloxy)phenyl]-3,7-dimethyl-2,4,-6,8-nonatetraensäure enthalten.

*Beispiel 24*

*Tablettenformulierung* (feuchte Granulierung)

| *Inhaltsstoffe* | *mg/Tablette* | | |
|---|---|---|---|
| 1. (all-E)-9-[2-nonyloxy)-phenyl]-3,7-dimethyl--2,4,6,8-nonatetraen-säure | 100 | 250 | 500 |
| 2. Lactose | 98,5 | 147,5 | 170 |
| 3. Polyvinylpyrrolidon (PVP) | 15 | 30 | 40 |
| 4. modifizierte Stärke | 15 | 30 | 40 |
| 5. Maisstärke | 15 | 30 | 40 |
| 6. Magnesiumstearat | 1,5 | 2,5 | 5 |
| Gewicht der Tablette | 245 mg | 490 mg | 795 mg |

*Verfahren:*

1) Mischen der Inhaltsstoffe 1, 2, 4 und 5. Granulieren mit PVP und Lösen in Wasser/Alkohol. Trocknen des Granulats. Vermahlen des trockenen Granulats.
2) Zusatz von Magnesiumstearat und Verpressen.

*Beispiel 25*

Nach dem in Beispiel 24 beschriebenen Verfahren werden Tabletten mit (all-E)-9-[2-Fluor-6-(nonyloxy)phenyl]-3,7-dimethyl-2,4,6,8-nonatetraensäure als aktiven Wirkstoff hergestellt.

*Beispiel 26*

*Tablettenformulierung* (direkte Verpressung)

| *Inhaltsstoffe* | *mg/Tablette* | | |
|---|---|---|---|
| 1. (all-E)-9-[2-(nonyl-oxy)-phenyl]-3,7-dimethyl-2,4,6,8-nonatetraensäure | 15 | 30 | 60 |
| 2. Lactose | 207 | 192 | 162 |
| 3. Avicel | 45 | 45 | 45 |
| 4. Stärke zur direkten Verpressung | 30 | 30 | 30 |
| 5. Magnesiumstearat | 3 | 3 | 3 |
| Gewicht der Tablette | 300 mg | 300 mg | 300 mg |

*Verfahren:*

1) Mischen von Inhaltsstoff 1 mit der gleichen Menge Lactose.
2) Zumischen der Inhaltsstoffe 3 und 4 und der verbleibenden Menge 1.
3) Zusatz von Magnesiumstearat und Mischen während 3 Minuten.
4) Verpressen.

*Beispiel 27*

*Kapselformulierung:*

| *Inhaltsstoff* | *mg/Kapsel* | | |
|---|---|---|---|
| 1. (all-E)-(3,7-Dimethyl)-9-[2-[(8-hydroxyoctyl)-oxy]-phenyl]-2,4,6,8--nonatetraensäure | 15 | 30 | 60 |
| 2. Lactose | 239 | 224 | 194 |
| 3. Stärke | 30 | 30 | 30 |
| 4. Talk | 15 | 15 | 15 |
| 5. Magnesiumstearat | 1 | 1 | 1 |
| Kapselfüllgewicht | 300 mg | 300 mg | 300 mg |

*Verfahren:*

1) Mischen der Stoffe 1-3.
2) Zusatz von Talk und Magnesiumstearat.
3) Verkapseln.

*Beispiel 28*

*Tablettenformulierung* (feuchte Granulierung)

| *Inhaltsstoff* | *mg/Tablette* | | |
|---|---|---|---|
| 1. (all-E)-(3,7-Dimethyl)-9-[2-(8-hydroxyoctyl)-oxy]-phenyl]-2,4,6,8-nonatetraensäure | 100 | 250 | 500 |
| 2. Lactose | 98,5 | 147,5 | 170 |
| 3. Polyvinylpyrrolidon | 15 | 30 | 40 |
| 4. Modifizierte Stärke | 15 | 30 | 40 |
| 5. Maisstärke | 15 | 30 | 40 |
| 6. Magnesiumstearat | 1,5 | 2,5 | 5 |
| Gewicht der Tablette | 245 mg | 490 mg | 795 mg |

*Verfahren:*

1) Mischen von 1, 2, 4 und 5, Granulieren mit PVP und
Lösen in Wasser/Alkohol. Trocknen des Granulats.
Mahlen des trockenen Granulats.
2) Zusatz von Magnesiumstearat und Verpressen.

*Beispiel 29*

Ein Gemisch von 51 g α,α,α-Trifluor-m-kresol, 70 g Bromnonan und 100 g Kaliumcarbonat in Dimethylformamid wurde 48 Stunden auf 85° erwärmt. Zusatz von Wasser und Hexan lieferte 89 g reinen (3-Trifluormethyl)phenyl-nonyläther, Siedepunkt 115°/13,3 Pa. Dieses Produkt wurde in 1,5 l Äther bei −20° mit 233 ml 1,5M n-Butyllithium in Hexan gemischt und 2 Stunden bei Raumtemperatur gerührt. Das Gemisch wurde dann auf −40° gekühlt, mit 40 ml trockenem Dimethylformamid in 100 ml Äther versetzt, auf 0° erwärmt und dann mit Wasser behandelt. Extraktion mit Hexan und Chromatographie auf Silicagel (5% Äther-Hexan) lieferte 35 g (2-Trifluormethyl-6-nonyloxy)benzaldehyd. Reduktion dieses Produkts mit Natriumborhydrid in Äthanol nach dem Verfahren von Beispiel 1 lieferte 32 g (2-Trifluormethyl-6-nonyloxy)benzolmethanol nach Chromatographie über Silicagel. 31 g dieses Materials wurden durch Umsetzung mit Triphenylphosphinhydrobromid nach dem Verfahren von Beispiel 1 in [[2-Trifluormethyl-6-(nonyloxy)phenyl]methyl]-triphenylphosphoniumbromid übergeführt.

*Beispiel 30*

97 mMol [[2-Trifluormethyl-6-(nonyloxy)phenyl]-methyl]-triphenylphosphoniumbromid in 600 ml Tetrahydrofuran wurde mit 7-Formyl-3-methyl-2,4,6--octatriensäureäthylester nach dem in Beispiel 3 beschriebenen Verfahren zu (all-E)-9-[2-(Trifluormethyl)-6-(nonyloxy)phenyl]-3,7-dimethyl-2,4,6,8--nonatetraensäureäthylester umgesetzt. Reinigung durch Chromatographie und Kristallisation aus Hexan lieferte den reinen Äthylester in 41% Ausbeute. 5,2 g Ester wurden wie in Beispiel 5 hydrolysiert und lieferten 3 g reine (all-E)-9-[2-(Trifluormethyl)-6--nonyloxy)phenyl]-3,7-dimethyl-2,4,6,8-nonatetraensäure. Schmelzpunkt 135-136° (aus Äthylacetat-Hexan).

*Beispiel 31*

2-Hydroxybenzaldehyd und 1-Bromhexan wurden nach dem Verfahren von Beispiel 1 zu [[2-(Hexyloxy)phenyl]methyl]triphenylphosphoniumbromid umgesetzt und diese in (all-E)-9-[2-(Hexyloxy)-phenyl]-3,7-diäthyl-2,4,6,8-nonatetraensäure, Schmelzpunkt 137-138° (aus Äthanol) nach dem Verfahren von Beispiel 3 übergeführt.

*Beispiel 32*

Eine Lösung von 1 Mol 4-Bromphenol in 500 ml Tetrahydrofuran wurde bei 25° zu einer Aufschlämmung von 1,17 Mol Natriumhydrid in 1,2 l Dimethylformamid gegeben. Nach vollständiger Reaktion wurden 1,32 Mol Allylchlorid zugesetzt und das Produkt nach 3 Stunden Rühren bei 45° mit Wasser und Hexan isoliert. Destillation lieferte Allyl-(4-bromphenyl)-äther. Siedepunkt 65-67°/13,3 Pa in 82% Ausbeute. Dieses Material wurde 4 Stunden mit Dimethylanilin auf 195° erhitzt und dann destilliert. Man erhielt 0,81 Mol 2-Allyl-4-bromphenol. Eine Lösung dieses Materials in 200 ml Tetrahydrofuran wurde bei 25° zu einem Gemisch von 0,8 Mol 1-Bromnonan, 0,92 Mol Natriumhydrid und 1 g Kaliumjodid in 1 l Dimethylformamid gegeben. Nach-

dem die Wasserstoffentwicklung beendet war, wurde das Gemisch 14 Stunden auf 50° erwärmt, gekühlt, in überschüssiges Wasser gegeben und mit Hexan extrahiert. Destillation lieferte 256 g Nonyl-(2-allyl-4-bromphenyl)äther, Siedepunkt 147-156°/13,3 Pa. 255 g dieses Materials in 1 l Dimethylsulfoxid und 0,5 l Tetrahydrofuran wurden 2 Stunden mit 2 g Kalium-t-butoxid auf 35-40° erwärmt und danach mit 5 ml Essigsäure und Wasser versetzt. Isolierung der Reaktionsprodukte mit Hexan lieferte 234 g reines 1-[2-(Nonyloxy)-5-(brom)phenyl]propen, Siedepunkt 145-155°/13,3 Pa. 0,56 Mol dieses Materials in 600 ml Tetrahydrofuran wurden mit 1 Mol Magnesium durch 3stündige Reaktion bei 55° in das Grignard-Reagens übergeführt. Nach Beendigung der Reaktion wurde auf 0° gekühlt und 0,75 Mol Trimethylborat in 200 ml Äther zugesetzt. Nach 30 Minuten Rühren bei 25° wurde das Gemisch auf 0° gekühlt, zu 500 ml eines Gemisches von 10% Ammoniumchloridlösung und 10% Wasserperoxid gegeben und 1 Stunde bei 25° gerührt. Nach Zusatz von Wasser wurde mit Hexan extrahiert und das Hexan unter vermindertem Druck abgedampft. Das Rohprodukt wurde über Silicagel filtriert und lieferte 73 g p-[2-(1-Propenyl)-4-(nonyloxy)phenyl]phenol. Acetylierung von 0,8 g dieses Materials mit Acetylchlorid und Triäthylamin in Dichlormethan lieferte 89% [2-(1-Propenyl)-4-(nonyloxy)-1-(acetoxy)]benzol. 99 g dieses Materials wurden in einem Gemisch von 150 ml Methanol und 1,5 Dichlormethan gelöst und bei −40° mit Ozon behandelt, bis das gesamte Ausgangsmaterial verbraucht war. Danach wurden 50 ml Dimethylsulfid und 500 ml Wasser zugesetzt, 30 Minuten kräftig bei 25° gerührt, die organische Phase über Magnesiumsulfat getrocknet und eingeengt. Man erhielt 83 g [2-(Nonyloxy)-5-(acetoxy)]benzaldehyd. Reduktion von 80 g dieses Materials mit 6 g Natriumborhydrid in 1 l Äthanol bei 20° während 2 Stunden lieferte rohes [2-(Nonyloxy)-5-(acetoxy)]benzolmethan, das sogleich 30 Minuten lang bei 60° mit 300 ml 40% wässrigem Kaliumhydroxid in 1 l Äthanol behandelt wurde. Ansäuern mit 6 M Salzsäure und Extraktion mit Chloroform liefert nach Einengen das Rohprodukt. Behandlung des Rückstandes mit Hexan lieferte 63 g [3-(Hydroxymethyl)-4-(nonyloxy)]phenol als Feststoff. Eine Lösung von 62 g dieses Materials in einem Gemisch von 0,5 l Acetonitril und 86 g Triphenylphosphinhydrobromid wurde 14 Stunden zum Rückfluss erhitzt und bei 50° zur Trockene eingeengt. Man erhielt [[2-(Nonyloxy)-5-(hydroxy)phenyl]methyl]triphenylphosphiniumbromid als Glas.

*Beispiel 33*

0,23 Mol [[2-(Nonyloxy)-5-(hydroxy)phenyl]methyl]triphenylphosphoniumbromid in 1,5 l tetrahydrofuran wurden bei −70° mit 315 ml 1,6M n-Butyllithium in Hexan und dann mit 59 g 8-Formyl-3,7-dimethyl-2,4,6-octatriensäureäthylester in Tetrahydrofuran umgesetzt. Das Gemisch wurde dann auf −15° erwärmt, mit Essigsäure angesäuert und mit Äther und 40%igen wässrigem Methanol extrahiert. Reinigung durch Chromatographie an Silicagel lieferte reinen (all-E)-9-[5-Hydroxy-2-(nonyloxy)phenyl]-3,7-dimethyl-2,4,6,8-nonatetraensäureäthylester. Hydrolyse von 6 g dieses Esters nach dem Verfahren von Beispiel 5 lieferte 3,5 g (all-E)-9-[5-Hydroxy-2-(nonyloxy)phenyl]-3,7-dimethyl-2,4,6,8-nonatetraensäure, Schmelzpunkt 170-173° (aus Äthylacetat).

*Beispiel 34*

4,4 g (all-E)-9-[5-Hydroxy-2-(nonyloxy)phenyl]-3,7-dimethyl-2,4,6,8-nonatetraensäureäthylester wurden mit 7 g Kaliumcarbonat, 6 g 2,2,2-Trifluoräthyl-p-toluolsulfonat in 200 ml Dimethylformamid 72 Stunden auf 90° erwärmt. Aufarbeitung mit Wasser und Hexan und anschliessende Reinigung über Silicagel lieferte 0,75 g reinen Äthylester. Hydrolyse von 0,9 g des Esters nach dem Verfahren von Beispiel 5 lieferte 0,6 g reine (all-E)-9-[2-(Nonyloxy)-5-(2,2,2-trifluoräthoxy)phenyl]-3,7-dimethyl-2,4,6,8-nonatetraensäure, Schmelzpunkt 121° (aus Tetrahydrofuran und Hexan).

*Beispiel 35*

Ein (E,Z)-Gemisch von 2-(1-Decenyl)-1-brombenzol (1:4), wie in Beispiel 16 hergestellt, wurde nach dem Verfahren von Beispiel 17 in ein (E,Z)-Gemisch von 2-(1-Decenyl)-1-hydroxymethylbenzol übergeführt. Das Gemisch wurde durch Chromatographie an Silicagel in die reinen (E)- und (Z)-Alkohole aufgetrennt. Umsetzung jedes dieser Isomeren mit Triphenylphosphinhydrobromid, wie in Beispiel 1, lieferten die entsprechenden Phosphoniumsalze, d.h. (Z)-[[2-(1-Decenyl)phenyl]methyl]triphenylphosphoniumbromid und (E)-[[2-(1-Decenyl)phenyl]methyl]triphenylphosphoniumbromid.

*Beispiel 36*

(E)-[[2-(1-Decenyl)phenyl]methyl]triphenylphosphoniumbromid wurde in den Äthylester der (all-E)-9-[2-(Decenyl)-phenyl]-3,7-dimethyl-2,4,6,8-nonatetraensäure nach dem in Beispiel 1 beschriebenen Verfahren übergeführt. Basische Hydrolyse, wie in Beispiel 1, und Kristallisation der rohen Säure aus Acetonitril lieferte (all-E)-9-[2-(1-Decenyl)-phenyl]-3,7-dimethyl-2,4,6,8-nonatetraensäure, Schmelzpunkt 105-107°.

*Beispiel 37*

(E,E,E,E,Z)-9-[2-(1-Decenyl)phenyl]-3,7-dimethyl-2,4,6,8-nonatetraensäure wurde in der gleichen Weise wie in Beispiel 36 unter Verwendung von (Z)-[[2-(1-Decenyl)phenyl]-methyl]triphenylphosphoniumbromid hergestellt. Hydrolyse des Äthylesters und Kristallisation der rohen Säure aus Äther lieferte reine (E,E,E,E,Z)-9-[2-(1-Decenyl)phenyl]-3,7-dimethyl-2,4,6,8-nonatetraensäure vom Schmelzpunkt 103-105°.

In den folgenden Beispielen ist die Verbindung A all(E)-9-[2-(Nonyloxy)phenyl]-3,7-dimethyl-2,4,6,8-nonatetraensäure. Diese Verbindung wurde in verschiedenen Testmodellen auf anti-inflammatorische Aktivität geprüft.

In allen Tests wurde die Verbindung A und die anderen geprüften Retinoide in Formulierungen in Erdnussöl angewandt, die 0,05% Propylgallat als Antioxidans enthielten. Die Dosis-Volumina waren

5 ml/kg für Ratten und 10 ml/kg für Mäuse. Kontrollgruppen wurden mit dem entsprechenden Volumen Erdnussöl behandelt.

*Beispiel 38*

Wirkung der Verbindung A auf die verzögerte Hypersensivität gegenüber methyliertem Rinderserumalbumin (MBSA).

*Versuchstiere:* Männliche und weibliche MFI-Mäuse. Anfangsgewicht etwa 25 g.

*Reagenzien:* Methyliertes Rinderserumalbumin (MBSA) Sigma; Freund's komplettes Adjuvans (Difco).

*Versuchsführung:* Mäuse wurden in Gruppen von 10, am Versuchstag 0, durch intradermale Injektion von 0,05 ml Wasser in Ölemulsion von MBSA und Freund's kompletten Adjuvans an zwei abdominalen Stellen sensibilisiert. Am Versuchstag 9 erhielten die Tiere eine Injektion von 20 µl 1%iger MBSA-Lösung in eine Pfote und 20 µl Wasser in die kontralaterale Pfote. Die Pfotenvolumina wurden 24 Stunden später plethysmographisch gemessen. Für jede Gruppe wurde die mittlere prozentuale Zunahme des Pfotenvolumens der mit MBSA behandelten Pfote im Vergleich zu der mit Wasser behandelten Pfote berechnet. Die Behandlung mit der Testsubstanz bzw. der Trägerlösung wurde am Tag 0 begonnen und am Tag 9 beendet.

Die Resultate sind in der Tabelle II angegeben.

TABELLE II

| | Dosis mg/kg | % Zunahme des Pfotenvolumens | % Reduktion gegenüber Kontrollen | mittlere Änderung des Körpergewicht(g) |
|---|---|---|---|---|
| Erdnussöl | 10 | 109 ± 11 | | M 3,8<br>F 0,2 |
| Etretinat | 10 | 59 ± 9** | 46 | M -0,8<br>F -2,0 |
| Verbindung A | 10 | 102 ± 12$^{ns}$ | 6 | M 3,3<br>F -0,2 |
| Verbindung A | 30 | 50 ± 7*** | 54 | M 2,8<br>F 0,5 |
| Verbindung A | 100 | 40 ± 5*** | 63 | M 3,3<br>F -0,2 |

Jede Gruppe umfasste vier männliche (M) und sechs weibliche (F) Mäuse, die in separaten Käfigen gehalten wurden. Die Verbindungen wurden oral mit einem Dosisvolumen von 10 ml/kg (10 Dosen) verabreicht.

ns = nicht signifikant
** = $p < 0,01$
*** = $p < 0,001$ (Student's T-Test).

*Beispiel 39*

Wirkung der Verbindung A auf Adjuvans-Arthritis bei der Ratte.

*Versuchstiere:* AHH/R-weibliche Ratten (Anfangsgewicht 110-140 g).

*Testsubstanzen:* Adjuvans zur Injektion. Eine homogenisierte Suspension hitzeabgetöteter M. tuberculosis (menschlicher Stamm C, DT und PN), 5 mg/ml in flüssigem Paraffin.

*Versuchsführung:* Die Ratten erhielten in Gruppen zu fünf subplantarer Injektionen von 0,1 ml Adjuvanssuspension in die rechte hintere Pfote. Die Versuchsverbindungen wurden am Morgen durch Intubation verabreicht, wobei mit dem Tag der Adjuvans-Injektion begonnen wurde. Zwei Kontrollgruppen und eine Gruppe von drei normalen Ratten (für Vergleichszwecke) erhielten Trägerlösung. Die Medikation wurde täglich durchgeführt, bis zum Versuchsende am 15. Tag, mit Ausnahme des ersten Wochenendes (Tage 5 und 6). Die Behandlungsgruppen sind in Tabelle III aufgeführt. Etretinat wurde als Standardretinoid verwendet.

Messungen des rechten hinteren Pfotenvolumens wurden zu Anfang und den Tagen 2 und 4 (primäre Phase) vorgenommen. Das rechte und hintere Pfotenvolumen wurde am Tag 8 und alle zwei oder drei Tage bis zum Ende des Versuchs am Tag 15 (sekundäre Phase) gemessen. Zu diesem Zeitpunkt wurde die Mobilität jedes Fussgelenks und die Häufigkeit und Schwere sekundärer Läsionen an Nase, Ohren, Vorderpfote, linkere hintere Pfote und Schwarz im Hinblick auf die Biegbarkeit unter Anwendung eines Punktesystems bewertet.

*Resultate:* Die Zeitverlaufskurven für die Pfoten mit Injektionen wurden für die Tage 0 bis 4 integriert als Massstab für die primäre Schwellung und vom Tag 8 bis 15 für die sekundäre Schwellung. Die sekundäre Schwellung in der Pfote, die keine Injektion erhalten hatte, wurde ähnlich zwischen den Versuchstagen 8 und 15 integriert. Die Resultate sind in Tabelle III zusammengestellt.

TABELLE III

| Behandlung | Dosis mg/kg p.o. | % Reduktion der Schwellung[1] der Pfote primär rechts | sekundär rechts | sekundär links | Läsionen[2] | Gelenkmobilität[1] | Gewichtsänderung (g) Tag 0-15 | Tag 8-15 |
|---|---|---|---|---|---|---|---|---|
| Normale Kontrollgruppe | — | — | — | — | — | — | +13,3*** | +3,7*** |
| Adjuvans-kontrollgr. | — | — | — | — | — | — | -0,6 | -5,5 |
| Verbindung A | 15 | -6 | 30 | 94*** | 72* | 66** | +2,2 | -0,4 |
| | 45 | 16 | 51*** | 75** | 65* | 66** | +5,4 | +1,2* |
| | 15 | 9 | 30 | 67* | 56* | 50 | +1,0 | -5,0 |

1. Students 't'-Test
2. Wilcoxon rank sum test
* = $P < 0,02$ *** = $P < 0,01$.

*Beispiel 40*

Wirkung der Verbindung A auf Typ II Collagen-Arthritis.

*Versuchstiere:* Männliche und weibliche Alderley Park Ratten.

*Versuchsmaterial:* Typ 2 Collagen (hergestellt aus Rindernasenseptumknorpel), Freund's inkomplettes Adjuvans (Difco).

*Versuchsführung:* Die Ratten wurden gegen Typ 2 Collagen sensibilisiert durch intradermale Injektion von 1 ml einer Wasser in Öl-Emulsion aus gleichen Teilen einer 1 mg/ml-Lösung Typ 2 Collagen in 0,45M NaCl, 0,02M Tris, pH 7,4 und Freund's inkomplettem Adjuvans. Die Ratten, die Arthritis entwickelten, wurden am Tag 15 nach Sensibilisierung zusammengefasst. Neben dieser Gruppe wurde eine mit Erdnussöl behandelte Kontrollgruppe (6 männliche, 4 weibliche Tiere) und eine mit der Verbindung A behandelte Gruppe (6 männliche, 5 weibliche Tiere) geführt. Die Volumina der hinteren Pfote wurden gemessen, um eine gleichmässige Verteilung der Tiere zwischen den Gruppen sicherzustellen. Zwischen den Tagen 15 und 16 wurde über Nacht der Urin gesammelt. Die Medikation begann am Tag 16. Urinproben wurden auf Glycosaminoglycane (GAG) analysiert. Die Verbindung A wurde mit 100 mg/kg p.o. dosiert. Vom Tag 19 auf den Tag 20 wurde über Nacht erneut der Urin gesammelt. Die Urinproben wurden auf Glycosaminoglycane analysiert. Am Tag 20 wurde eine zweite Messung der hinteren Pfote vorgenommen. Die Ratten wurden dann mit Natriumpentobarbiton anaesthesiert und ausgeblutet und es wurden Röntgenaufnahmen der Hinter- und Vorderpfoten vorgenommen. Die Ratten erhielten das Versuchspräparat in 4 Dosen an den Tagen 16-19. Die Resultate sind in der Tabelle IV zusammengestellt.

TABELLE IV

| Tag | Verbindung A 100 mg/kg$^{-1}$ Pfotenvolumen | GAG (µg) | Gewicht (g) | Kontrollen Erdnussöl Pfotenvolumen | GAG (µg) | Gewicht (g) |
|---|---|---|---|---|---|---|
| 16 | 2,44 ± 0,08 | 1586 ± 307 | 247 ± 14 | 2,48 ± 0,07 | 1538 ± 192 | 249 ± 19 |
| 20 | 2,66 ± 0,06 | 634 ± 72 | 229 ± 10 | 2,46 ± 0,07 | 729 ± 134 | 258 ± 19 |

*Beispiel 41*

Wirkung der Verbindung A auf nicht-immunogene Entzündung.

*Versuchstiere:* Weibliche Alderley Park-Ratten im Gewicht von 170-205 g.

*Versuchsmaterialien:* Lambda-Carrageenan, hergestellt als Lösung in Kochsalzlösung und sterilisiert.

*Versuchsführung:* Die Verbindung A wurde während 10 Tagen einmal täglich oral an Ratten in Gruppen von 8 in Dosierungen von 10, 30 und 100 mg/kg verabreicht. Die Kontrolltiere erhielten den Träger. Eine Stunde nach der letzten Dosis wurden die Tiere mit Methohexiton anaesthesiert und 0,2 ml 1%iges Lambda-Carrageenan wurde in die Pleuralhöhle injiziert. 4 Stunden später wurden die Tiere durch eine Überdosis von Pentobarbiton getötet, das Pleuralexudat wurde gesammelt und die Pleuralhöhle mit 2 ml Phosphat-gepufferter Kochsalzlösung ausgewaschen. Das Exudat-Volumen wurde gemessen und die Zellen mit einem automatischen Zähler bestimmt. Differentialzählbestimmungen wurden an Exudatabstrichen nach Giemsa-Färbung vorgenommen, um die Zahl der mehrkernigen Leukozyten (PMN) und einkernigen Zellen (MN) einzeln zu bestimmen.

Unmittelbar nach Gewinnung des Pleuralexudats wurden die Tibiae der Tiere entfernt und deren Bruchlast festgestellt. Die Körpergewichte der Tiere wurden täglich ermittelt. Die Resultate sind in Tabelle V zusammengestellt.

TABELLE V

| Verbindung | Dosis (p.o.) | Exudat-volumen | Gesamte Zellzahl | PMN-Zellen | MN-Zellen | Tibia-Bruch-belastung |
|---|---|---|---|---|---|---|
| | | (ml) % Verände-rung | $\times 10^6$ % Verände-rung | $\times 10^6$ % Verände-rung | $\times 10^6$ % Verände-rung | Rechts und links (kg) Mitte % Veränderung |
| Kontrolle Erdnussöl | 5 ml | 1,40 ±0,16 | 129,3 ±3,1 | 119,1 ±2,9 | 10,2 ±0,3 | 6,7 ±0,3 |
| Verbindung A (Lösung) | 10 mg | 0,94* -32,8 ±0,08 | $120,8^{N.S.}$ -6,5 ±7,3 | $112,4^{N.S.}$ -5,6 ±6,7 | $8,4^{N.S.}$ -17,4 ±0,8 | $6,5^{N.S.}$ -3,4 ±0,1 |
| Verbindung A | 30 mg | 0,88* -36,9 ±0,09 | 112,1* -13,3 ±6,0 | 104,2* -12,4 ±5,6 | 7,9* -23,1 ±0,7 | $6,8^{N.S.}$ +1,5 ±0,3 |
| Verbindung A | 100 mg | 0,80** -42,9 ±0,08 | $109,1^{N.S.}$ -15,7 ±8,1 | $101,6^{N.S.}$ -14,7 ±7,1 | 7,5* -26,7 ±1,1 | $6,7^{N.S.}$ -0,3 ±0,2 |

N.S. = nicht significant
* = p < 0,05; ** = < 0,01, im Vergleich zu Kontrolle (Student's 't'-Test.

*Beispiel 42*

Wirkung der Verbindung A im Schwammgranulom-Test bei der Ratte.

*Versuchstiere:* AHH/R weibliche Ratten mit einem Anfangsgewicht von 120-140 g.

*Versuchsmaterial:* Aus Celluloseschwammtuch wurden Pellets mit einem Durchmesser von 6,5 mm ausgestanzt. Auf jedes Pellet wurden 0,1 ml einer Suspension, die 0,5 mg/ml hitzeabgetötete M. tuberculosis-Bakterien (menschlicher Stamm C, Dt und PN) in steriler Kochsalzlösung enthielt, gegeben. Die Pellets wurden getrocknet, gewogen und sterilisiert.

*Versuchsführung:* Die Ratten wurden in Gruppen zu 5 aufgeteilt. Die Testverbindungen wurden täglich verabreicht. Nach der fünften Dosis wurden die Ratten anästhesiert, der Rücken rasiert und zwei Pellets wurden jeder Ratte auf jeder Seite dorsal implantiert. 7 Tage nach der Implantation wurden die Ratten getötet und die Pellets entfernt von anhängendem Gewebe befreit und gewogen. Jedes Pellet wurde dann in 4 ml destilliertes Wasser gegeben, fein zerkleinert und beschallt. Nach Zentrifugieren wurde der Natrium- und Kaliumgehalt des Überstands flammenphotometrisch bestimmt. Zusätzlich wurden Nebennieren und Thymusdrüsen jeder Ratte entfernt und gewogen und die hinteren Unterschenkel zur Bestimmung der Tibia-Bruchlast entfernt. Die Körpergewichte wurde ebenfalls im Verlauf des Testes bestimmt. Die Resultate sind in der Tabelle VI zusammengestellt.

TABELLE VI

| Behandlung | Dosis $mg/kg^{-1}$ | No. 1 Anzahl Ver-suchs-tiere | % Abnahme des Granulom-gewichts | | | % Veränderung von | | | Änderung des Körper-gewichts (g) |
|---|---|---|---|---|---|---|---|---|---|
| | | | feucht | Na+ | K+ | Neben-nieren-gewicht | Thymus-gewicht | Tibia-Bruch-last | |
| Kontrolle | — | 8 | — | — | — | — | — | — | +9,6±1,3 |
| Verbindung A | 15 p.o. | 5 | 15,1** | 14,5** | -6,9** | +17,6** | -4,4 | -4,0 | +7,2±0,74 |
| | 45 p.o | 5 | 10,8 | 11 | 0,8 | +29,0** | +14,3 | -1,12 | +11,4±0,98 |
| Etretinat | 15 p.o | 4 | 2,7 | 5,9 | -3,9 | +22,9** | +15,2 | -4,6 | +13,2±2,6 |
| Dexamethason | 0,5 sc | 5 | 65,5*** | 51,2*** | 75,2*** | -49,5*** | -79*** | -11,5* | -10,2±1,59 |

* p = < 0,05; ** p = < 0,02; *** p = < 0,01.

*Beispiel 43*

Wirkung auf dem Verlauf von Adjuvans-Arthritis.

*Versuchstiere:* Männliche Lewis-Ratten.

*Versuchsmaterial:* Hitzeabgetötetes getrocknetes Mycobacterium butyricum.

*Versuchsführung:* Die Adjuvans-Arthritis wurde durch Injektion von 0,1 ml Adjuvans (Suspension hitzeabgetöteter Mycobacterium butyricum 0,5% CW/V) in schwerem Mineralöl, das 0,2% Digitonin enthielt) in die Schwanzwurzeln erzeugt. Nach dem sich die Arthritis 21 Tage entwickelt hatte, wurde das Volumen beider Hinterpfoten plethysmographisch gemessen. Die Ratten wurden in Gruppen von 8 mit gleichen mittleren Pfotenvolumen aufgeteilt und dann mit Verbindung A, Indomethacin (als Kontrolle) oder Vehikel für die Dauer von 7 Tage behandelt. Danach wurden die Volumina beider Hinterpfoten erneut gemessen, um antiinflammatorische Effekte zu bestimmen. Gewichtsänderungen wurden ebenfalls ermittelt und am Versuchsende wurde Plasmafibrinogen (Amer. J. Clin. Path. 71: 521-527) ermittelt.

Die Resultate sind in der Tabelle VII angegeben.

TABELLA VII

| Gruppe | Behandlung | Dosis (mg/kg) | Änderung des Pfotenvolumens (ml) | Plasmafibrinogen (mg/dl) | Änderung des Körpergewichts (g) |
|---|---|---|---|---|---|
| Arthritic (10)[b] | Vehikel | — | +0,53±0,08 | 1773±30 | 7,5±1,2 |
| Arthritic (10) | Verbindung A | 100 | -0,96±0,10* | 874±57* | 5,2±2,1 |
| Arthritic (10) | Indomethacin | 1 | -1,22±0,15* | 978±100* | 25,6±3,1* |

[b] Verabreichung einmal täglich durch Intubation beginnend mit dem Tag 22 nach Induzierung der Arthritis unter Verwendung von Tween 80 als Vehikel.

[c] Differenz von Volumen oder Gewicht am Tag 28 und Volumen oder Gewicht am Tag 22. Beide Hinterpfoten wurden berücksichtigt.

* Signifikant verschieden vom Wert für arthritische Tiere die nur Vehikel erhalten hatten ($p < 0,05$).

**Patentansprüche**

1. Verbindung der Formel

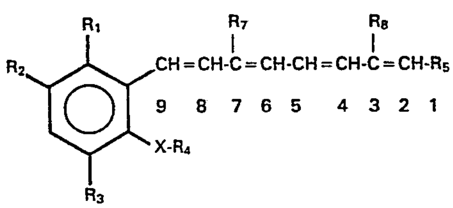

I

worin

$R_1$ Wasserstoff, Niederalkyl, Chlor, Fluor oder Trifluormethyl; $R_2$ Wasserstoff, Niederalkoxy, Trifluormethylniederalkoxy, Hydroxy, Niederalkyl, Chlor, Trifluormethyl oder Fluor; $R_3$ Wasserstoff, Niederalkyl, Chlor oder Fluor; $R_4$ Alkyl mit 4-10 C-Atomen oder $-CH_2(CH_2)_nOH$; X $-CH(R_{10})O-$, $-CH(R_{10})-$, -O-, $-C(R_{10})=CH-$ oder $-N(R_{10})-$; $R_5$ $COOR_9$; n 6 oder 7; und $R_7$, $R_8$, $R_9$ und $R_{10}$ Niederalkyl oder Wasserstoff bedeuten und Salze davon, wenn $R_9$ Wasserstoff ist.

2. Verbindungen gemäss Anspruch 1, in denen $R_1$ Wasserstoff, Chlor oder Fluor; $R_2$ Wasserstoff, nieder-Alkoxy, Chlor oder Fluor; $R_3$ Wasserstoff, nieder-Alkyl, Chlor oder Fluor; $R_4$ Alkyl mit 8-10 C-Atomen und 8 oder 9 C-Atomen in gerader Kette; X $-CH(R_{10})O-$, $-CH(R_{10})-$, -O- oder $-N(R_{10})-$ ist und n, $R_5$, $R_7$, $R_8$ und $R_{10}$ dasselbe wie in Anspruch 1 bedeuten; und Salze davon, wenn $R_5$ COOH ist.

3. Verbindungen gemäss Anspruch 1 oder 2, worin $R_4$ Alkyl mit 4-10 C-Atomen ist.

4. Verbindungen gemäss Anspruch 3, worin X--O- ist.

5. Verbindungen gemäss Anspruch 4, worin $R_1$ Chlor oder Fluor ist.

6. Verbindungen gemäss Anspruch 4, worin $R_2$ nieder-Alkoxy ist.

7. 9-[2-Chlor-6-(nonyloxy)phenyl]-3,7-dimethyl--2,4,6,8-nonatetraensäure.

8. (All-E)-9-[2-fluor-6-(nonyloxy)phenyl]-3,7-dimethyl-2,4,6,8-nonatetraensäure.

9. 3,7-Dimethyl-9-(5-methoxy-2-nonyloxy-phenyl)-2,4,6,8-nonatetraensäure.

10. 9-[2-Nonyloxy)phenyl]-3,7-dimethyl-2,4,-6,8-nonatetraensäure.

11. (All-E)-3,7-dimethyl-9-(2-octylaminophenyl)-2,4,6,8-nonatetraensäure.

12. (All-E)-3,7-dimethyl-9-[2-[(8-hydroxyoctyl)-oxy]phenyl]-2,4,6,8-nonatetraensäure.

13. (All-E)-8-[2-(trifluormethyl)-6-(nonyloxy)--phenyl]-3,7-dimethyl-2,4,6,8-nonatetraensäure,
3,7-Dimethyl-9-[2-(octyloxy)phenyl]-2,4,6,8-nonatetraensäure,
3,7-Dimethyl-9-[2-(2,2-dimethyl-octyl)oxy]-phenyl--[-2,4,6,8-nonatetraensäure,
9-[2-(Nonyloxyphenyl]-3,7-dimethyl-2,4,6,8-nonatetraensäure-äthylester,
(all-E)-9-[2-(hexyloxy)phenyl]-3,7-dimethyl-2,4,-6,8-nonatetraensäure,

(all-E)-9-[5-Hydroxy-2-(nonyloxy)phenyl]-3,7-dimethyl-2,4,6,8-nonatetraensäure,
(all-E)-9-[2-(Nonyloxy)-5-(2,2,2-trifluoräthoxy)phenyl]-3,7-dimethyl-2,4,6,8-nonatetraensäure,
3,7-Dimethyl-9[2-[(octyloxy)-methyl]-phenyl]-2,4,-6,8-nonatetraensäure,
9-(Decylphenyl)-3,7-dimethyl-2,4,6,8-nonatetraensäure,
3,7-Dimethyl-9-(2-octylamino-phenyl)-2,4,6,8--nonatetraensäure-äthylester und
(all-E)-9-[2-(1-Decenyl)phenyl]-3,7-dimethyl-2,4,-6,8-nonatetraensäure.

14. Verbindungen der Formel I und Salze davon, wenn $R_9$ Wasserstoff ist, zur Verwendung als antirheumatische, anti-arthritische und immunsuppressive Mittel.

15. 9-[2-(Nonyloxy)phenyl]-3,7-dimethyl-2,4,-6,8-nonatetraensäure zur Verwendung als antirheumatische, anti-arthritische und immunosuppressive Mittel.

16. Verbindung der Formel I und Salze davon, wenn $R_9$ Wasserstoff ist, zur Verwendung bei der Herstellung von anti-rheumatischen, anti-arthritischen und immunsuppressiven Mitteln.

17. 9-[2-(Nonyloxy)phenyl]-3,7-dimethyl-2,4,-6,8-nonatetraensäure zur Verwendung bei der Herstellung von anti-rheumatischen, anti-arthritischen und immunsuppressiven Mitteln.

18. Pharmazeutische Präparate, enthaltend eine Verbindung der Formel I gemäss Anspruch 1 und übliche pharmazeutische Trägerstoffe.

19. Pharmazeutische Präparate enthaltend 9-[2--(Nonyloxy)phenyl]-3,7-dimethyl-2,4,6,8-nonatetraensäure und übliche pharmazeutische Trägerstoffe.

20. Verfahren zur Herstellung von Verbindungen der Formel

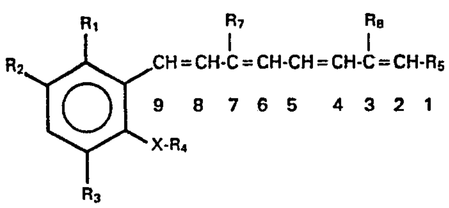

I

worin

$R_1$ Wasserstoff, Niederalkyl, Chlor, Fluor oder Trifluormethyl; $R_2$ Wasserstoff, Niederalkoxy, Trifluormethylniederalkoxy, Hydroxy, Niederalkyl, Chlor, Trifluormethyl oder Fluor; $R_3$ Wasserstoff, Niederalkyl, Chlor oder Fluor; $R_4$ Alkyl mit 4-10 C-Atomen oder $-CH_2(CH_2)_nCH_2OH$; X $-CH(R_{10})O-$, $-CH(R_{10})-$, $-O-$, $-C(R_{10})=CH-$ oder $-N(R_{10})-$; $R_5$ $COOR_9$; n 6 oder 7; und $R_7$, $R_8$, $R_9$ und $R_{10}$ Niederalkyl oder Wasserstoff bedeuten und Salze davon, wenn $R_9$ Wasserstoff ist, dadurch gekennzeichnet, dass man

a) eine Verbindung der Formel

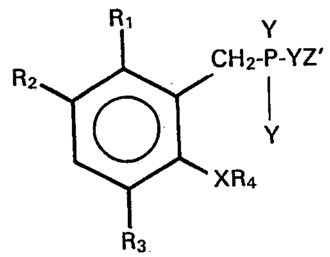

II

mit einer Verbindung der Formel

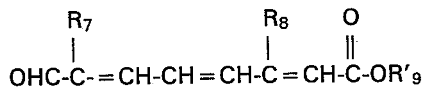

VII

oder

b) eine Verbindung der Formel

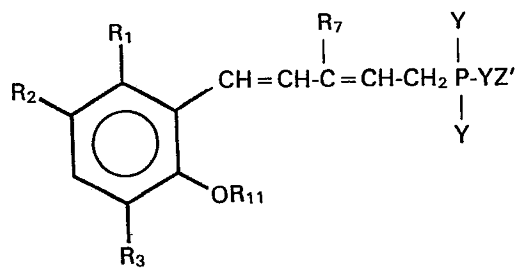

XVI

mit einer Verbindung der Formel

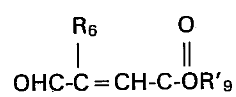

XVII

oder

c) eine Verbindung der Formel

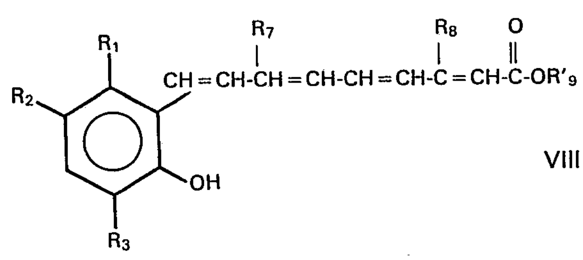

VIII

mit einer Verbindung der Formel $R_4$ Z umsetzt, wobei in den obigen Formeln $R_1$, $R_2$, $R_3$, $R_4$, $R_7$ und $R_8$ die in Anspruch 1 angegebene Bedeutung haben, $R_9'$ nieder-Alkyl; Y Aryl, Z eine Abgangsgruppe und Z' ein Halogenidion ist; und dass man gewünschtenfalls eine Gruppe $-COOR_9'$ im Reaktionsprodukt in die freie Säure überführt und diese gewünschtenfalls in ein Salz überführt.

21. Verbindungen der Formel

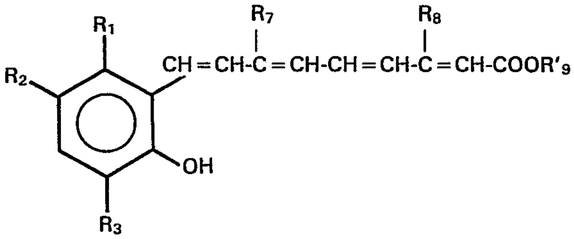

worin $R_1$, $R_2$, $R_3$, $R_7$ und $R_8$ die in Anspruch 1 angegebene Bedeutung haben und $R_9'$ nieder-Alkyl ist.

22. Verbindungen der Formel

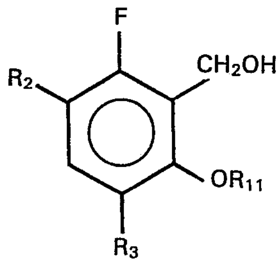

worin $R_2$ und $R_3$ die in Anspruch 1 angegebene Bedeutung haben und $R_{11}$ eine Alkylgruppe mit 8-10 C-Atomen oder eine Gruppe $-CH_2(CH_2)_nCH_2OR_{17}$ ist; n 6 oder 7 und $-OR_{17}$ Hydroxy oder eine in eine Hydroxygruppe überführbare Äthergruppe ist.

23. Verbindungen der Formel

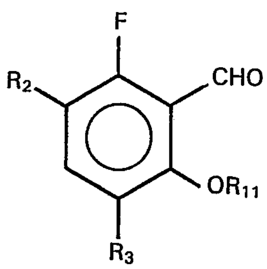

worin $R_2$, $R_3$ und $R_{11}$ die in Anspruch 1 bzw. 22 angegebene Bedeutung haben.

24. Verbindungen der Formel

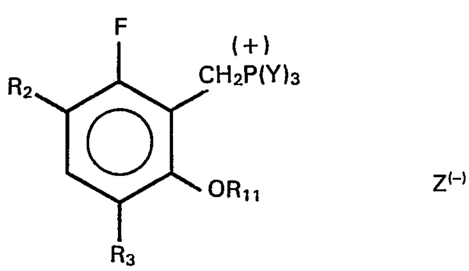

worin $R_2$, $R_3$ und $R_{11}$ die in Anspruch 1 bzw. 22 angegebene Bedeutung haben; Y Aryl und $Z^{(-)}$ ein Halogenidion ist.

**Claims**

1. Compounds of the formula

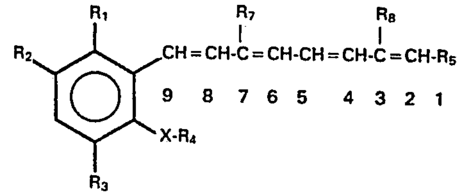

I

wherein $R_1$ signifies hydrogen, lower alkyl, chlorine, fluorine or trifluoromethyl; $R_2$ signifies hydrogen, lower alkoxy, trifluoromethyl-lower alkoxy, hydroxy, lower alkyl, chlorine, trifluoromethyl or fluorine; $R_3$ signifies hydrogen, lower alkyl, chlorine or fluorine; $R_4$ signifies alkyl with 4-10 C-atoms or $-CH_2(CH_2)_nCH_2OH$; X signifies $-CH(R_{10})O-$, $-CH(R_{10})-$, -O-, $-C(R_{10})=CH-$ or $-N(R_{10})-$; $R_5$ signifies $COOR_9$; n signifies 6 or 7; and $R_7$, $R_8$, $R_9$ and $R_{10}$ signify lower alkyl or hydrogen
and salts thereof when $R_9$ is hydrogen.

2. Compounds in accordance with claim 1, in which $R_1$ is hydrogen, chlorine or fluorine; $R_2$ is hydrogen, lower alkoxy, chlorine or fluorine; $R_3$ is hydrogen, lower alkyl, chlorine or fluorine; $R_4$ is alkyl with 8-10 C-atoms and with 8 or 9 C-atoms in a straight-chain; X is $-CH(_{10})O-$, $-CH(R_{10})-$, -O- or $-N(R_{10})-$ and n, $R_5$, $R_7$, $R_8$ and $R_{10}$ signify the same as in claim 1; and salts thereof when $R_5$ is COOH.

3. Compounds in accordance with claim 1 or 2, wherein $R_4$ is alkyl with 4-10 C-atoms.

4. compounds in accordance with claim 3, wherein X is -O-.

5. Compounds in accordance with claim 4, wherein $R_1$ is chlorine or fluorine.

6. Compounds in accordance with claim 4, wherein $R_2$ is lower alkoxy.

7. 9-[2-Chloro-6-(nonyloxy)phenyl]-3,7-dimethyl-2,4,6,8-nonatetraenoic acid.

8. (All-E)-9-[2-fluoro-6-(nonyloxy)phenyl]-3,7--dimethyl-2,4,6,8-nonatetraenoic acid.

9. 3,7-Dimethyl-9-(5-methoxy -2-nonyloxyl-phenyl)-2,4,6,8-nonatetraenoic acid.

10. 9-[2-Nonyloxy)phenyl]-3,7-dimethyl-2,4,6,8--nonatetraenoic acid.

11. (All-E)-3,7-dimethyl-9-(2-octylaminophenyl)-2,4,6,8-nonatetraenoic acid.

12. (All-E)-3,7-dimethyl-9-[2-[(8-hydroxyoctyl)-oxyphenyl]-2,4,6,8-nonatetraenoic acid.

13. (All-E)-8-[2-(trifluoromethyl)-6-(nonyloxy)--phenyl]-3,7-dimethyl-2,4,6,8-nonatetraenoic acid,
3,7-dimethyl-9-[2-(octyloxy)phenyl]-2,4,6,8-nonatetraenoic acid,
3,7-dimethyl-9-[2-(2,2-dimethyl-octyl)oxy]-phenyl-[2,4,6,8-nonatetraenoic acid,
9-[2-(nonyloxyl-phenyl)]-3,7-dimethyl-2,4,6,8--nonatetraenoic acid,
(all-E)-9-[2-(hexyloxy)phenyl]-3,7-dimethyl-2,4,-6,8-nonatetraenoic acid,

(all-E)-9-[5-hydroxy-2-(nonyloxy)-phenyl]-3,7-dimethyl-2,4,6,8-nonatetraenoic acid,
(all-E)-9-[2-nonyloxy)-5-(2,2,2-trifluoroethoxy)phenyl]-3,7-dimethyl-2,4,6,8-nonatetraenoic acid,
3,7-dimethyl-9[2-[(octyloxy)-methyl]-phenyl]-2,4,-6,8-nonatetraenoic acid,
9-(decylphenyl)-3,7-dimethyl-2,4,6,8-nonatetraenoic acid,
3,7-dimethyl-9-(2-octylaminophenyl)-2,4,6,8--nonatetraenoic acid ethyl ester and
(all-E)-9-[2-(1-decenyl)phenyl]-3,7-dimethyl-2,4,-6,8-nonatetraenoic acid.

14. Compounds of formula I and salts thereof when $R_9$ is hydrogen for use as anti-rheumatic, anti-arthritic and immunosuppressive agents.

15. 9-[2-(Nonyloxy)phenyl]-3,7-dimethyl-2,4,-6,8-nonatetraenoic acid for use as an anti-rheumatic, anti-arthritic and immunosuppressive agent.

16. Compounds of formula I and salts thereof when $R_9$ is hydrogen for use in the manufacture of anti-rheumatic, anti-arthritic and immunosuppressive medicaments.

17. 9-[2-(Nonyloxy)phenyl]-3,7-dimethyl-2,4,-6,8-nonatetraenoic acid for use in the manufacture of anti-rheumatic, anti-arthritic and immunosuppressive medicaments.

18. Pharmaceutical preparations containing a compound of formula I in accordance with claim 1 and usual pharmaceutical carrier materials.

19. Pharmaceutical preparations containing 9-[2--(nonyloxy)phenyl]-3,7-dimethyl-2,4,6,8-nonatetraenoic acid and usual pharmaceutical carrier materials.

20. A process for the manufacture of compounds of the formula

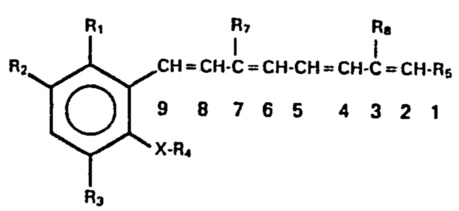

I

wherein $R_1$ signifies hydrogen, lower alkyl, chlorine, fluorine or trifluoromethyl; $R_2$ signifies hydrogen, lower alkoxy, trifluoromethyl-lower alkoxy, hydroxy, lower alkyl, chlorine, trifluoromethyl or fluorine; $R_3$ signifies hydrogen, lower alkyl, chlorine or fluorine; $R_4$ signifies alkyl with 4-10 C-atoms or $-CH_2(CH_2)_nCH_2OH$; X signifies $-CH(R_{10})O-$, $-CH(R_{10})-$, -O-, $-C(R_{10})=CH-$ or $-N(R_{10})-$; $R_5$ signifies $COOR_9$; n signifies 6 or 7; and $R_7$, $R_8$, $R_9$ and $R_{10}$ signify lower alkyl or hydrogen
and salts thereof when $R_9$ is hydrogen, characterized by

a) reacting a compound of the formula

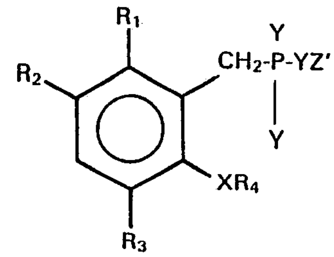

II

with a compound of the formula

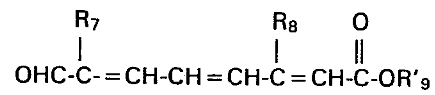

VII

or

b) reacting a compond of the formula

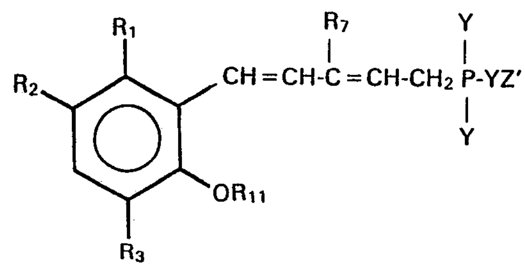

XVI

with a compound of the formula

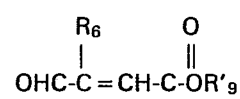

XVII

or

c) reacting a compound of the formula

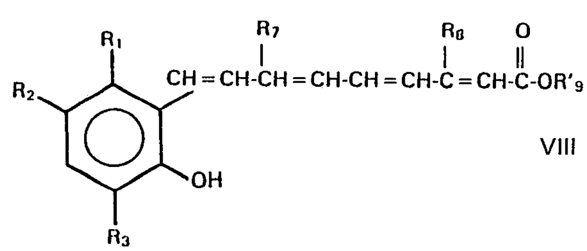

VIII

with a compound of the formula $R_4Z$, wherein in the above formulae $R_1$, $R_2$, $R_3$, $R_4$, $R_7$ and $R_8$ have the significance given in claim 1, $R_9$, is lower alkyl; Y is aryl, Z is a leaving group and Z' is a halogenide ion; and, if desired, converting a group $-COOR_9$, in the reaction product into the free acid and, if desired, converting this into a salt.

21. Compounds of the formula

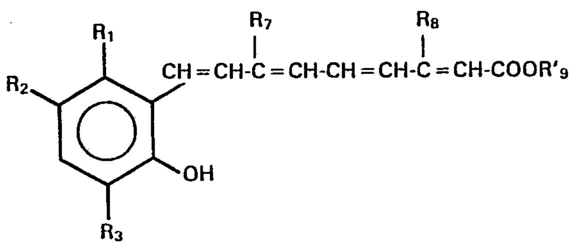

wherein $R_1$, $R_2$, $R_3$, $R_7$ and $R_8$ have the significance given in claim 1 and $R_9$, is lower alkyl.

22. Compounds of the formula

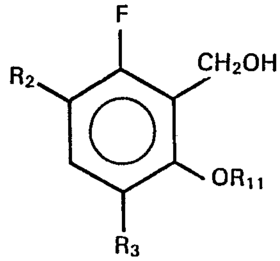

wherein $R_2$ and $R_3$ have the significance given in claim 1 and $R_{11}$ is an alkyl group with 8-10 C-atoms or a group $-CH_2(CH_2)_nCH_2OR_{17}$ ; n is 6 or 7 and $-OR_{17}$ is hydroxy or an ether group convertible into a hydroxy group.

23. Compounds of the formula

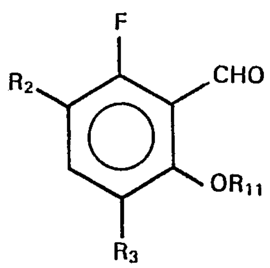

wherein $R_2$, $R_3$ and $R_{11}$ have the significance given in claim 1 or 22.

24. Compounds of the formula

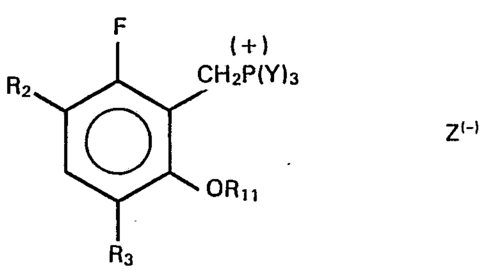

wherein $R_2$, $R_3$ and $R_{11}$ have the significance given in claim 1 or 22; Y is aryl and $Z^{(-)}$ is a halogenide ion.

**Revendications**

1. Composé de formule

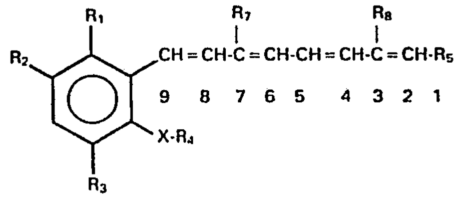

I

dans laquelle $R_1$ représente l'hydrogène, un groupe alkyle inférieur, le chlore, le fluor ou un groupe trifluorométhyle; $R_2$ représente l'hydrogène, un groupe alcoxy inférieur, trifluorométhyl-alcoxy inférieur, hydroxy, alkyle inférieur, le chlore, un groupe trifluorométhyle ou le fluor; $R_3$ représente l'hydrogène, un groupe alkyle inférieur, le chlore ou le fluor; $R_4$ représente un groupe alkyle en C4-C10 ou un groupe $-CH_2(CH_2)_nCH_2OH$; X représente $-CH(R_{10})O-$, $-CH(R_{10})-$, -O-, $-C(R_{10})=CH-$ ou $-N(R_{10})-$; $R_5$ représente $COOR_9$; n est égal à 6 ou 7, et $R_7$, $R_8$, $R_9$ et $R_{10}$ représentent des groupes alkyle inférieurs ou l'hydrogène, et leurs sels lorsque $R_9$ représente l'hydrogène.

2. Composés selon la revendication 1, dans lesquels $R_1$ représente l'hydrogène, le chlor ou le fluor; $R_2$ représente l'hydrogène, un groupe alcoxy inférieur, le chlore ou le fluor; $R_3$ représente l'hydrogène, un groupe alkyle inférieur, le chlore ou le fluor; $R_4$ représente un groupe alkyle en C8-C10 avec 8 ou 9 atomes de carbone en chaîne droite; X représente $-CH(R_{10})O-$, $-CH(R_{10})-$, -O- ou $-N(R_{10})-$ et n, $R_5$, $R_7$, $R_8$ et $R_{10}$ ont les significations indiquées dans la revendication 1; et leurs sels lorsque $R_5$ représente COOH.

3. Composés selon la revendication 1 ou 2, dans lesquels $R_4$ représente un groupe alkyle en C4-C10.

4. Composés selon la revendication 3, dans lesquels X représente -O-.

5. Composés selon la revendication 4, dans lesquels $R_1$ représente le chlore ou le fluor.

6. Composés selon la revendication 4, dans lesquels $R_2$ représente un groupe alcoxy inférieur.

7. L'acide 9-[2-chloro-6-(nonyloxy)-phényl]-3,7-diméthyl-2,4,6,8-nonatétraénoïque.

8. L'acide (tout-E)-9-[2-fluoro-6-(nonyloxy)-phényl]-3,7-diméthyl-2,4,6,8-nonatétraénoïque.

9. L'acide 3,7-diméthyl-9-(5-méthoxy-2-nonyloxy-phényl)-2,4,6,8-nonatétraénoïque.

10. L'acide 9-[2-nonyloxy)phényl]-3,7-diméthyl-2,4,6,8-nonatétraénoïque.

11. L'acide (tout-E)-3,7-diméthyl-9-(2-octylaminophényl)-2,4,6,8-nonatétraénoïque.

12. L'acide (tout-E)-3,7-diméthyl-9-[2-[(8-hydroxyoctyl)-oxy]-phényl]-2,4,6,8-nonatétraénoïque.

13. L'acide (tout-E)-8-[2-(trifluorométhyl)-6-(nonyloxy)phényl]-3,7-diméthyl-2,4,6,8-nonatétraénoïque,

l'acide 3,7-diméthyl-9-[2-(octyloxy)-phényl]-2,4,-6,8-nonatétraénoïque,

l'acide 3,7-diméthyl-9-[2-(2,2-diméthyl-octyl)-oxy]-phényl]-2,4,6,8-nonatétraénoïque,
le 9-[2-(nonyloxy)phényl]-3,7-diméthyl-2,4,6,8-nona-tétraénoate d'éthyle,
l'acide (tout-E)-9-[2-(hexyloxy)-phényl]-3,7-diméthyl-2,4,6,8-nonatétraénoïque,
l'acide (tout-E)-9-[5-hydroxy-2-(nonyloxy)-phényl]-3,7-diméthyl-2,4,6,8-nonatétraénoïque,
l'acide (tout-E)-9-[2-(nonyloxy)-5-(2,2,2-trifluoréthoxy)-phényl]-3,7-diméthyl-2,4,6,8-nonatétraénoïque,
l'acide 3,7-diméthyl-9-[2-[(octyloxy)-méthyl]-phényl]-2,4,6,8-nonatétraénoïque,
l'acide 9-(décylphényl)-3,7-diméthyl-2,4,6,8-nonatétraénoïque,
le 3,7-diméthyl-9-(2-octylaminophényl)-2,4,6,8-nonatétraénoate d'éthyle et
l'acide (tout-E)-9-[2-(1-décényl)-phényl]-3,7-diméthyl-2,4,6,8-nonatétraénoïque.

14. Composés de formule I et, lorsque $R_9$ représente l'hydrogène, leurs sels, pour l'utilisation en tant qu'agents anti-rhumatismaux, anti-arthritiques et immuno-suppressifs.

15. L'acide 9-[2-(nonyloxy)-phényl]-3,7-diméthyl-2,4,6,8-nonatétraénoïque pour l'utilisation en tant qu'agent anti-rhumatismal, anti-arthritique et immuno-suppressif.

16. Composé de formule I et, lorsque $R_9$ représente l'hydrogène, ses sels, pour l'utilisation dans la préparation de produits anti-rhumatismaux, anti-arthritiques et immuno-suppressifs.

17. L'acide 9-[2-(nonyloxy)-phényl]-3,7-diméthyl-2,4,6,8-nonatétraénoïque pour l'utilisation dans la préparation de produits anti-rhumatismaux, anti-arthritiques et immuno-suppressifs.

18. Compositions pharmaceutiques contenant un composé de formule I selon la revendication 1 et des véhicules pharmaceutiques usuels.

19. Compositions pharmaceutiques contenant de l'acide 9-[2-(nonyloxy)-phényl]-3,7-diméthyl-2,4,6,8-nonatétraénoïque et des véhicules pharmaceutiques usuels.

20. Procédé de préparation des composés de formule

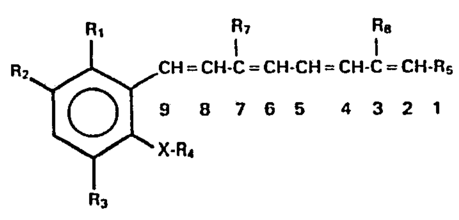

I

dans laquelle $R_1$ représente l'hydrogène, un groupe alkyle inférieur, le chlore, le fluor ou un groupe trifluorométhyle; $R_2$ représente l'hydrogène, un groupe alcoxy inférieur, trifluorométhyl-alcoxy inférieur, hydroxy, alkyle inférieur, le chlore, un groupe trifluorométhyle ou le fluor; $R_3$ représente l'hydrogène, un groupe alkyle inférieur, le chlore ou le fluor; $R_4$ représente un groupe alkyle en C4-C10 ou $-CH_2(CH_{2n}-CH_2OH$; X représente $-CH(R_{10})O-$, $-CH(R_{10})-$, -O-, $-C(R_{10})=CH-$ ou $-N(R_{10})-$; $R_5$ représente $COOR_9$; n est égal à 6 ou 7 et $R_7$, $R_8$, $R_9$ et $R_{10}$ représentent des groupes alkyle inférieurs ou l'hydrogène,
et de leurs sels lorsque $R_9$ représente l'hydrogène, caractérisé en ce que:

a) on fait réagir un composé de formule

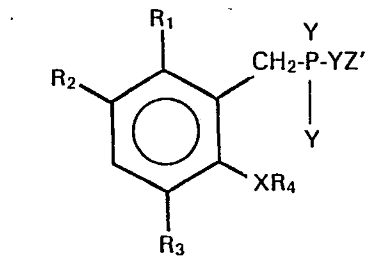

II

avec un composé de formule

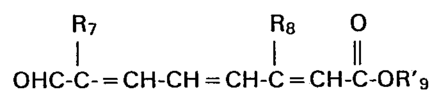

VII

ou bien

b) on fait réagir un composé de formule

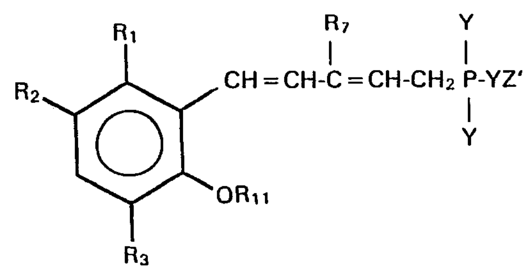

XVI

avec un composé de formule

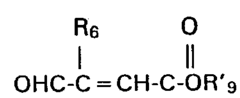

XVII

ou bien

c) on fait réagir un composé de formule

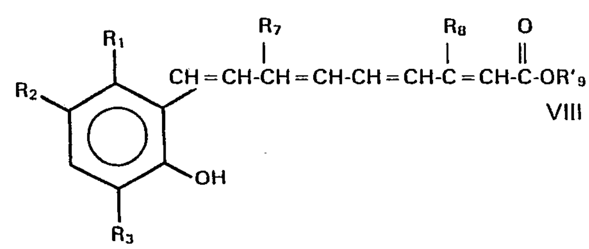

VIII

avec un composé de formule $R_4Z$, les symboles $R_1$, $R_2$, $R_3$, $R_4$, $R_7$ et $R_8$ ayant dans les formules ci-dessus les significations indiquées dans la revendication 1, $R'_9$ représente un groupe alkyle inférieur; Y représente un groupe aryle, Z un groupe éliminable et Z' un ion halogénure; et si on le désire, on convertit un groupe $-COOR'_9$ contenu dans le produit de réaction en l'acide libre et celui-ci, si on le désire, en un sel.

21. Composés de formule

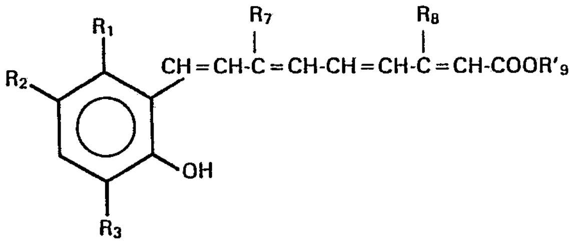

dans laquelle $R_1$, $R_2$, $R_3$, $R_7$ et $R_8$ ont les significations indiquées dans la revendication 1 et $R'_9$ représente un groupe alkyle inférieur.

22. Composés de formule

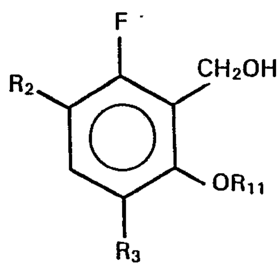

dans laquelle $R_2$ et $R_3$ ont les significations indiquées dans la revendication 1, et $R_{11}$ représente un groupe alkyle en C8-C10 ou un groupe $-CH_2(CH_2)_nCH_2OR_{17}$; n est égale à 6 ou 7 et $-OR_{17}$ représente un groupe hydroxy ou un groupe éther convertible en un groupe hydroxy.

23. Composés de formule

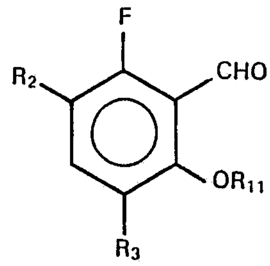

dans laquelle $R_2$, $R_3$ et $R_{11}$ ont les significations indiquées respectivement dans les revendications 1 et 22.

24. Composés de formule

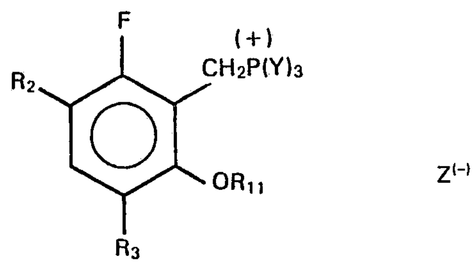

dans laquelle $R_2$, $R_3$ et $R_{11}$ ont les significations indiquées respectivement dans les revendications 1 et 22; Y représente un groupe aryle et $Z^{(-)}$ un ion halogénure.

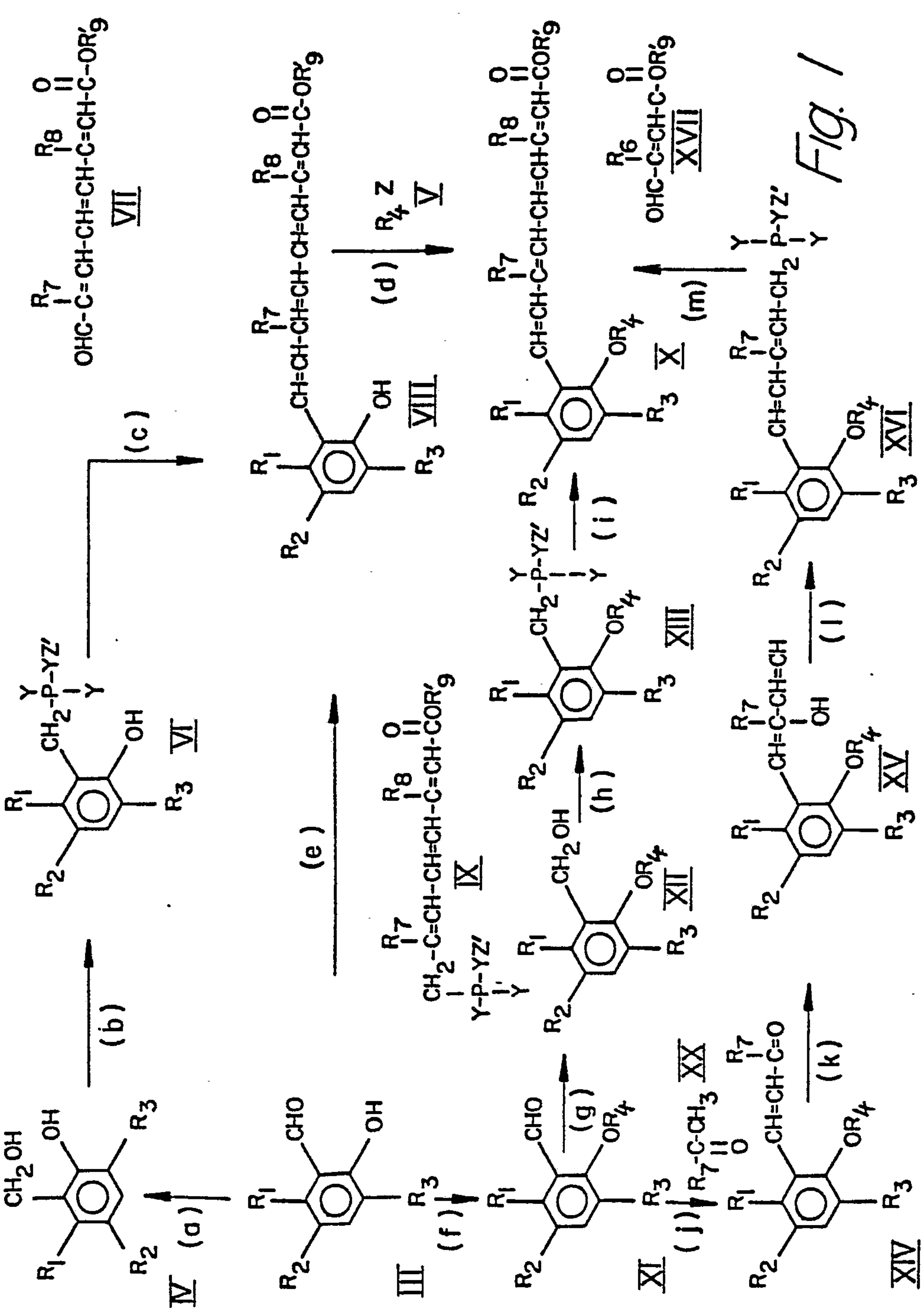

IV
III
XI
XIV
(a)
(b)
(c)
(d)
$R_4Z$ V
(e)
(f)
(g)
(h)
(i)
(j)
(k)
(l)
(m)
VI
VII
VIII
IX
X
XII
XIII
XV
XVI
XVII
XX


Fig. 1

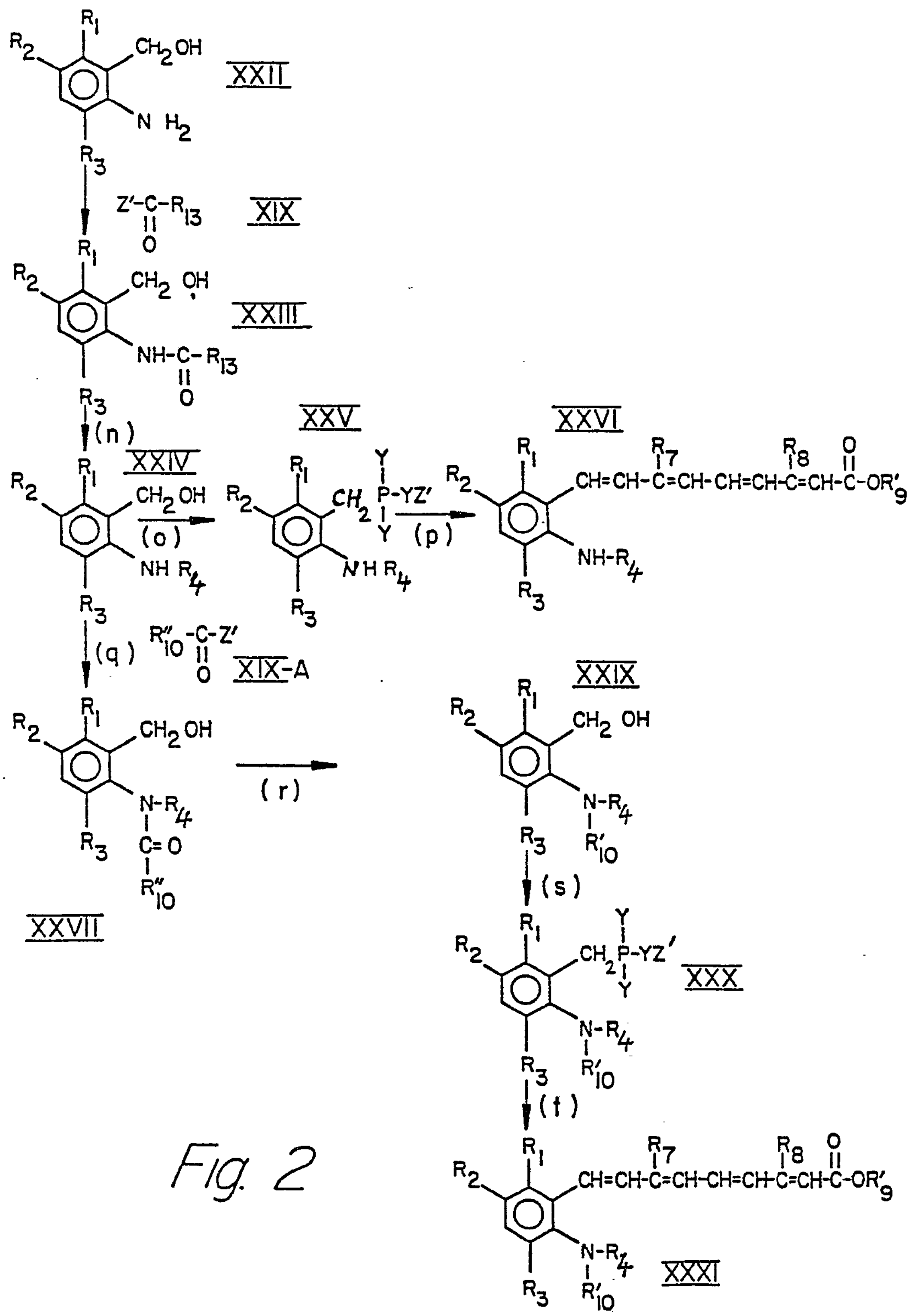


XXII XIX XXIII XXIV XXV XXVI XIX-A XXVII XXIX XXX XXXI

Fig. 2

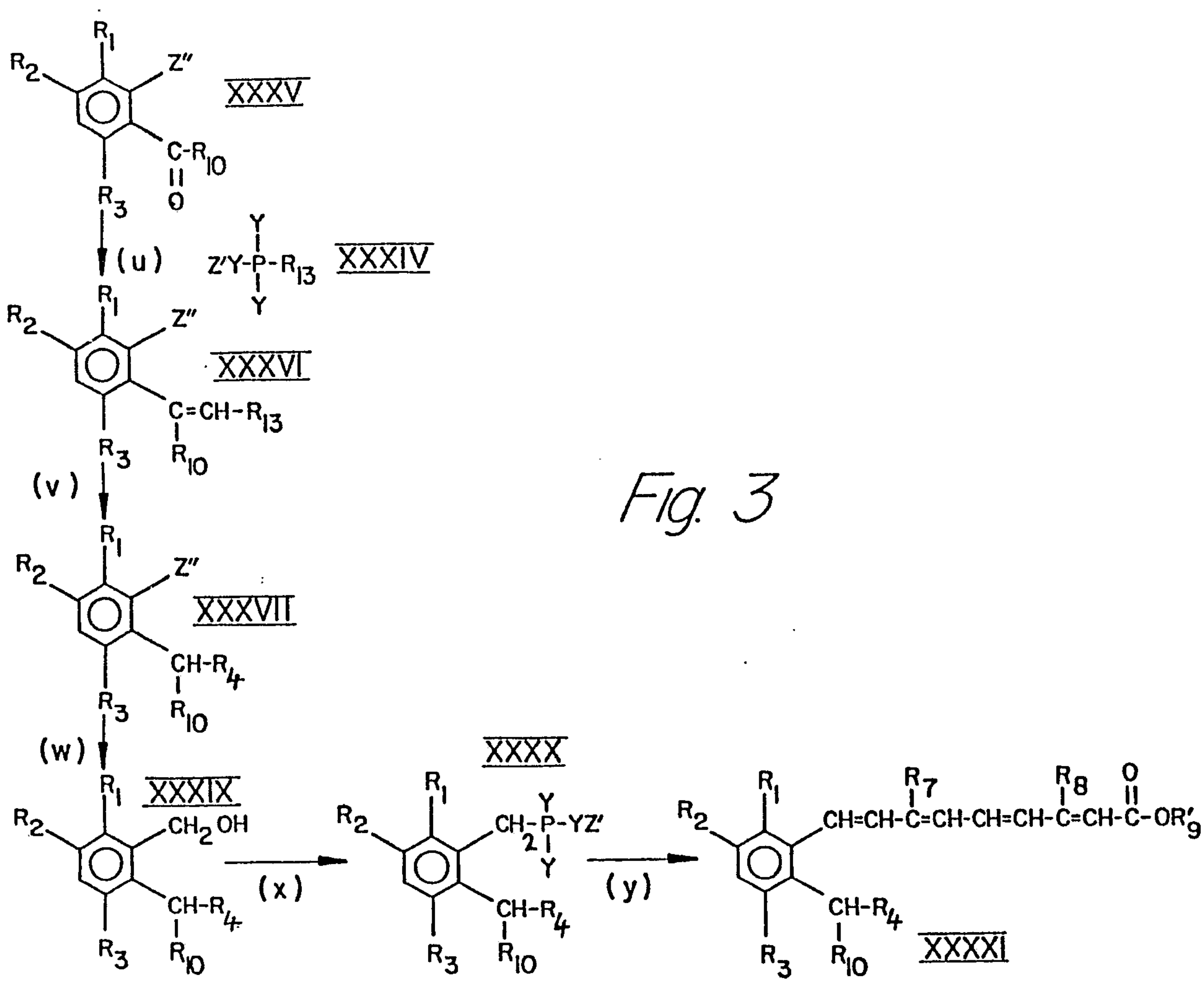


XXXV XXXIV XXXVI XXXVII XXXIX XXXX XXXXI

Fig. 3

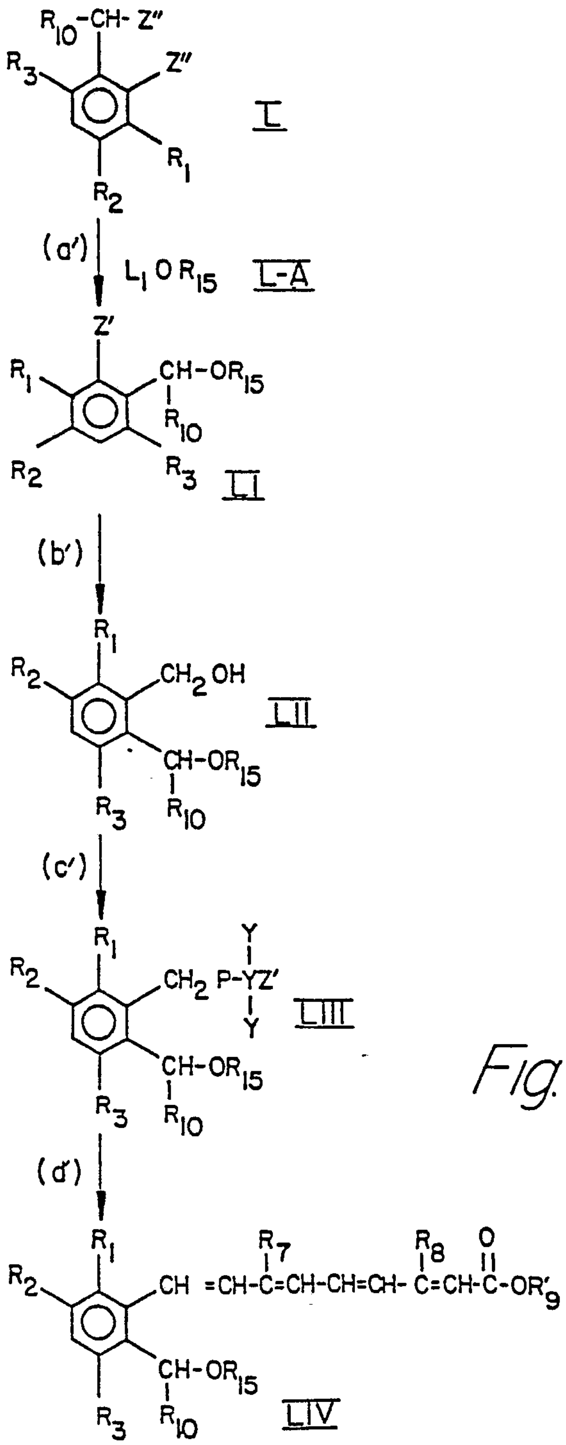


Fig. 4

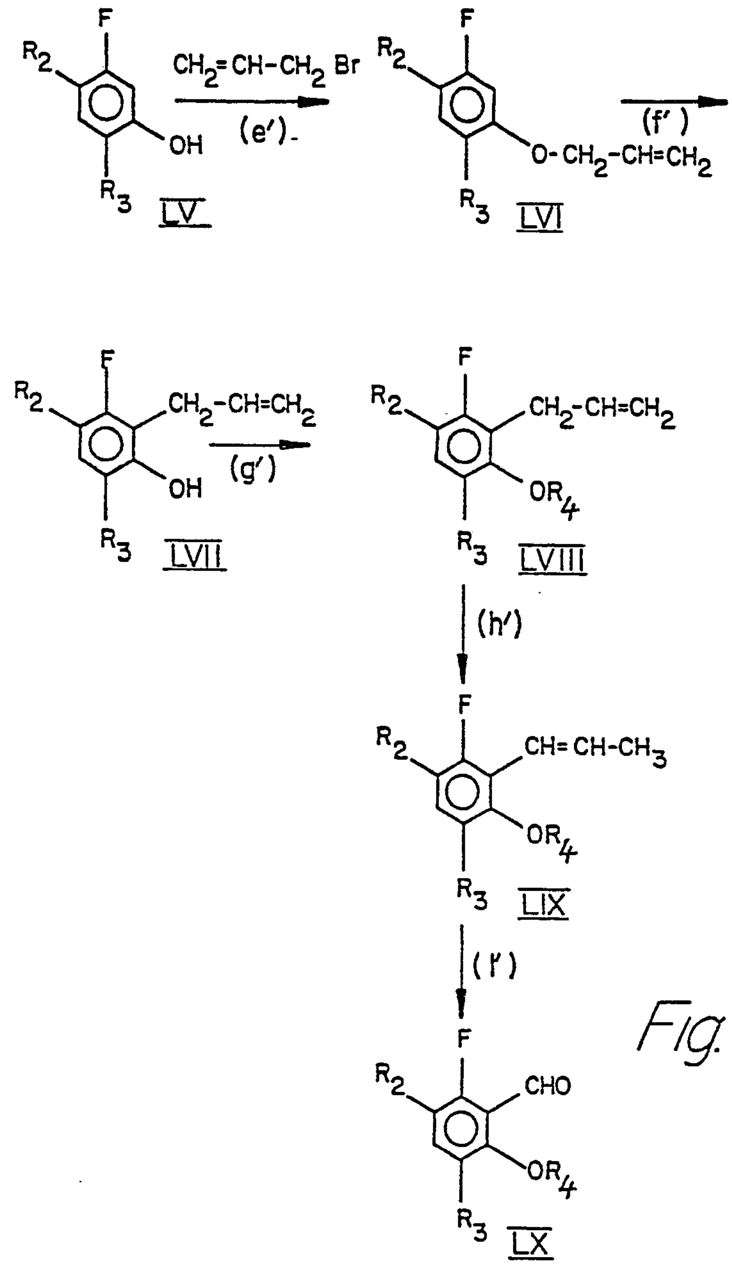


Fig. 5

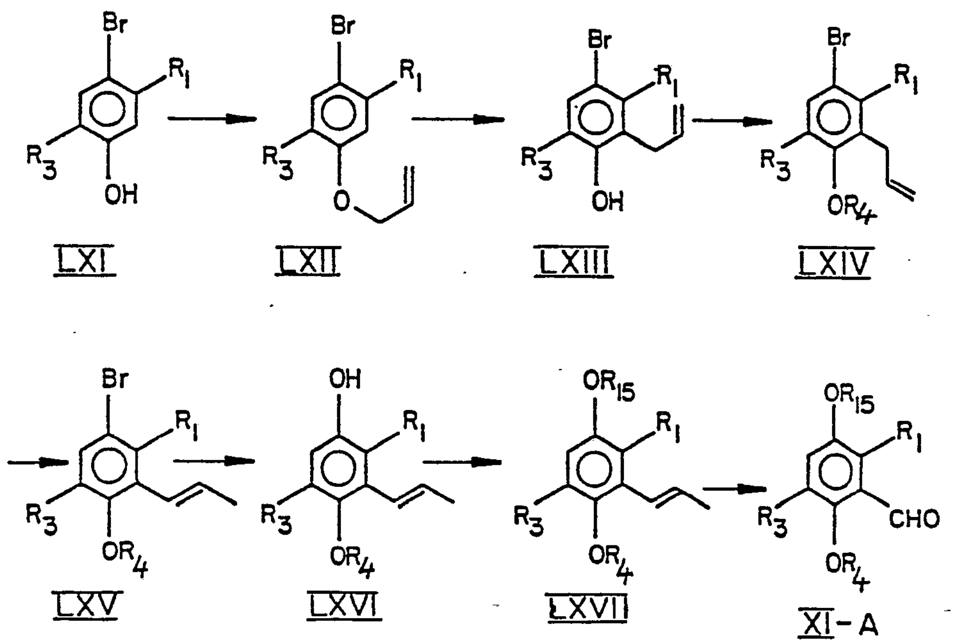

LXI LXII LXIII LXIV

LXV LXVI LXVII XI-A

Fig. 6

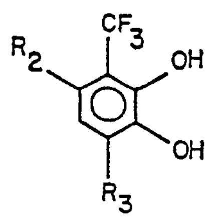

LXX

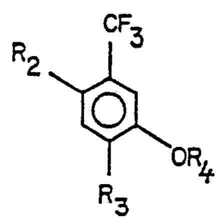

LXI

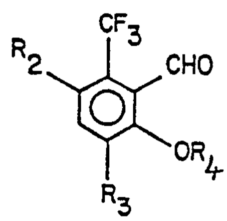

XI-B

*Fig. 7*